(19) Europäisches Patentamt / European Patent Office / Office européen des brevets

(11) **EP 3 888 665 A2**

(12) **EUROPEAN PATENT APPLICATION**
published in accordance with Art. 153(4) EPC

(43) Date of publication:
06.10.2021  Bulletin 2021/40

(51) Int Cl.:
*A61K 35/00* (2006.01)      *A61P 25/00* (2006.01)
*A61P 15/00* (2006.01)      *A61P 9/00* (2006.01)
*A61K 45/00* (2006.01)

(21) Application number: 19889970.0

(22) Date of filing: 25.11.2019

(86) International application number:
PCT/CN2019/120725

(87) International publication number:
WO 2020/108446 (04.06.2020 Gazette 2020/23)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority:  28.11.2018  CN 201811437559
25.08.2019  CN 201910787175

(71) Applicant: **Dong, Futian**
**Suzhou, Jiangsu 215123 (CN)**

(72) Inventor: **Dong, Futian**
**Suzhou, Jiangsu 215123 (CN)**

(74) Representative: **Habermann, Hruschka &**
**Schnabel**
**Patentanwälte**
**Montgelasstraße 2**
**81679 München (DE)**

(54) **SUBSTANCE FOR DISEASE TREATMENT AND/OR PREVENTION, AND DESIGN METHOD AND PREPARATION METHOD THEREFOR**

(57)  The present disclosure provides a substance for treatment and/or prevention of diseases, the method for designing the substance, and the method for preparing the substance. A stable structure corresponding to a substance associated with diseases is used as the substance for treatment or prevention of the diseases. The essence of disease is the imbalance of the biological structure system. The structural imbalance of different substances will cause different diseases. The present invention discloses that the biological body recognizes the stable structure corresponding to the substance, adjusts its gene or gene expression by the self-adaptation and self-organization functions of its own structural system, and restores the biological structure system to be related to the substance. The balance of structure, treatment and/or prevention of diseases related to the substance.

EP 3 888 665 A2

## Description

**Technical field**

The present disclosure relates to the technical field of medicaments, health foods, food additives and the like, and specifically relates to substances for treatment and/or prevention of diseases, the method for designing them, and the method for preparing them.

**Background Art**

According to a new medical model—*Structural information medicine*, the present invention provides understanding of life from the perspective of the *structure system*, and achieves the purpose of preventing and treating diseases by allowing the body to recognize specific structural information, and utilizing function, such as the self-adapting and self-organizing of the structure system of the body to restore the dynamic balance of the structure system.

1. Medical models of traditional Chinese medicine and Western medicine

Western medicine and traditional Chinese medicine are major existing medical systems. Western medicine is based on microscopic local structures of substances, and has been doing well in characterizing substances, but not so well in formulating general laws underlying substances, whereas the traditional Chinese medicine is mainly based on a general overall functional system, and has been doing well in formulating general laws underlying substances but not in characterizing them. The two medical systems complement each other, but have theories and research methods opposite to each other. Western medicine mainly relies on scientific experimentation to analyze the material nature of diseases by reductionism, by means of basic research methods such as anatomical physiology, biochemistry, molecular biology, and clinical trials. Therefore, Western medicine pays more attention to the pathologically changed structural substances, analyzes specific changes in structure, and directly curbs the excessive and remedies the deficient at a level of local structure of substances by means of chemical drugs, surgeries, radiation, or the like. For example, therapies such as H2 receptor antagonists for treatment of gastric hyperacidity and hormone replacement therapy take effect rapidly and have been highly effective in treatment of some acute diseases, surgical diseases and infectious diseases. However, since comprehensive understanding of the entire body has not been achieved in Western medicine, such therapies can only temporarily solve problems with a local structure, and long-term application thereof will have an adverse effect on other structures of the body and disrupt the balance in the body. In fact, since 2010, one third of diseases in the world are drug-induced diseases, which are caused by inevitable side effects of drugs. In addition, Western medicine has a mild effect on chronic degenerative diseases caused by complex pathogenic factors and influenced by multiple factors, such as cardiovascular and cerebrovascular diseases, multiple sclerosis, and senile dementia, and has difficulty in curbing the increasing morbidity and mortality due to these diseases.

Traditional Chinese medicine treats life as a whole, unifies people, society, psychology and environment, and reflects the objective laws of nature and people as well as health and disease, with *Bianzheng and Lunzhi* (identification of indication then treatment) as its central principle. In traditional Chinese medicine, the concept "health" refers to a relatively balanced, coordinated, and ordered state of the body's *Yin* and *Yang* in both subjective and objective environments, that is, the balance of the functional system. When this balance is disrupted, the order and homeostasis in the human body is disturbed and damaged, that is, the functional system of the human body is out of balance, and then diseases appear. Traditional Chinese medicine diagnoses the nature of diseases by consulting patients for a large amount of information about the functional system, and then promotes the body's functional system by means of drugs, acupuncture, massage, and the like. In general, it curbs the excessive, remedies the deficient, strengthens the body resistance (positive, ZHENG, or GOOD), eliminates the pathology (negative, XIE, or BAD), and promotes the positives, thereby restoring the homeostasis of the body. Traditional Chinese medicine takes effect slowly, but reduces the side effects of replacement therapies or antagonistic drugs, and has been quite effective in prevention and treatment of diseases, health preserving, and rehabilitation.

In summary, Western medicine focuses more on the microscopic structural balance, while traditional Chinese medicine pays more attention to the macroscopic functional system balance, both of which aim to correct the imbalance of the body but at different levels. However, they are completely different in epistemology and methodology about diseases and health. In order to take the benefits of both medical models, we need to integrate them with a more inclusive perspective and theory, so as to develop substances more effective in treatment of diseases and to significantly benefit human health.

*2. Structural information medicine*

(1) A fundamental theory that integrates the Western medicine and traditional Chinese medicine with the structure system

After the *Big Bang*, positive and negative charges appeared and interacted to form a substance of a simple structure, hydrogen, and then the interaction between the structures led to more complex structures, and finally a macroscopic substance, including the entire biological world. Therefore, all things in the universe are ascribed to the structures, and the substance, energy, and information that constitute the universe are also ascribed to the structures. Firstly, different substances are made of different structures, and substances are specific structures and the basis for functioning. The structure and function of a living system match each other, a specific structure corresponds to a specific function, and a change in specific structure will also naturally affect the specific functions of the corresponding system. Secondly, the bonding energy of the interaction between structures is a relatively abstract structure indicating stability of structure, and energy is the internal driving force for interconnection of structures. Finally, the relationship and order between structures is information, and information is the most abstract structure among the three, combines and governs substances and energy, and reflects the internal law and nature of structures. For example, the nature of an organism is determined by its abstract structure—genes. Therefore, structure is the unification of substances, energy and information, that is, the unification of concrete structures and abstract structures.

An organism is an organic system, essentially an organic structure system dominated by abstract structures. This structure system is an organic structural whole with a certain function formed by a relatively stable communication mode, the order of organization, and the temporal and spatial relationships between a number of elements. Any element itself is also a structure system, a sub-system that constitutes a part of the original system and certainly consists of sub-sub-systems and so on. The biological structure system is composed of structural sub-systems at different levels, such as the immune system and the gastrointestinal system. On the other hand, an organism is a sub-system of a larger structure system. For example, an organism is a sub-system of nature-a larger structure system, and maintains its own existence and reproduction in constant exchanges of substance, energy and information with nature.

DNA as a genetic material of organisms stores all information in the process of life activities, including growth, development, aging, and death. According to the *biochemical individuality* theory by Dr. Roger Williams, difference in biochemical composition between human bodies originates from the difference in genes and gene expressions, that is, from the difference in the molecular spatial structure of gene expression. Dr. Susumu Tonegawa, a Nobel laureate in physiology, stated that all diseases except trauma are associated with genes. The causes of diseases mainly include two aspects: (i) a structure constituting a part of the body is damaged or changed by the environment of the structure, or (ii) a gene is improperly expressed. The root cause of structural imbalance is a change in an abstract structure, that is, a change in gene expression, such as abnormal histone acetylation and methylation, abnormal gene methylation, etc. If a specific structure is changed, as long as the genes related to the structure can be expressed normally, the body's metabolism will gradually replace the pathologically changed structure with a normal structure, so that the body can return to a healthy state. Therefore, the key to the health of an organism lies in genes and gene expressions.

Western medicine studies the changes in specific structures, building models from diseases and using the models to treat diseases. Western medicine focuses more on studies of local tissue and functions in the structure system. With the assistance of instrument, detection can be made deep into specific and microscopic structures of substances. The form of existence of specific structures and morphologies of substances in the human body are given more attention and weight, such as anatomy, molecular biology, cell biology, histology and embryology, etc. These disciplines experimentally investigate and observe specific structures in the structure system from different angles, but have not done enough in abstract thinking and have overlooked the integrity of the body as a structure system. Therefore, it is easy to have limitations in understanding and practice. Western medicine mainly directly intervenes in specific structures by changing and influencing the local structure in the body with drugs to achieve a new balance, including research on genes, which also tends to directly change genes or gene expressions. Traditional Chinese medicine builds models from humans, and studies the functions of various organs of the body and the relationships and interactions between the functions under the guidance of holism. Traditional Chinese medicine does not detect specific structures of substances, but diagnoses diseases through manifestations of functions and external information. However, functions are determined by structures, and the functional system is essentially a structure system. The mutual promoting and restraining effects between functional sub-systems are essentially the mutual enhancing and inhibiting actions between the structural sub-systems. Traditional Chinese medicine achieves an overall balance by regulating the functional order and strength of the body, the essence of which is to restore the dynamic balance of the structure system. However, due to lack of an in-depth understanding of specific structures in the body, traditional Chinese medicine also has great limitations. Western medicine mainly establishes disease models from the perspective of microscopic structures, while traditional Chinese medicine establishes life models from the perspective of macroscopic functions. The microscopic structures found by Western medicine provide a material basis for indications of traditional Chinese medicine, that is, all the indications of traditional Chinese medicine have a material support from gene expression, i.e., a structural support, and are essentially due to a microscopic structural imbalance. In the structure system, based on the unification of the microscopic and the macroscopic, parts and a whole, and the concrete and the abstract, the specific structures may be converted into abstract structural information by utilization of local and microscopic concrete structures and recognition of the concrete structures by the body, thereby stimulating the body's self-adapting and self-organizing function to regulate genes or gene expressions and regain the balance of the structure system.

As a system, the structure system has basic characteristics such as system integrity, openness, stability, and self-organization. Relative to the environment, the system is unification of enclosures and openness. In the structure system, the interaction among structures is based on the interaction between the structures and the environment as a proviso, and on the external environment of the structures as a condition. The evolution of the same structure system depends on the external structure. Proper changes and adjustments of gene expressions according to changes in the external structure is a fundamental function of an organism to adapt to the environment, maintain its survival, and maintain its own health. If this function is absent, it will generally become extinct. The structure system of the human body has this powerful health function. By structural information of specific structures, Structural information medicine aims to mobilize and modulate the relatively complex, highly ordered, and self-regulated mechanism of action in the structure system, including sub-systems at different levels, such as genes, molecules, cells, tissues and organs, so as to restore the body's structural balance and achieve the purpose of preventing and treating diseases. It not only integrates the Western medicine's theory focusing on specific structures and functions as research objects, but also integrates traditional Chinese medicine's theory focusing on restoration of balance by regulating the body itself.

(2) Problems to be solved by Structural information medicine

Living organisms cannot exist independent of the environment. The external structural environment interacts with the body's structure system through structures, namely substance, energy and information. Each individual is a unique structure system resulting from both genes and environment. Structural information medicine focuses on the dynamic balance between the internal structure system in an organism and its external structural environment. It is this dynamic balance that interprets the process of life. In addition to restoring the dynamic balance of the body's own structure system and enhancing the body's resistance, structural information medicine further gives weight to symbiosis and co-progress. Symbiosis means that the body no longer blindly combats and defends itself against external structures, but constantly internalizes the external structural information, and achieves a balance between the its structure system and the external structure through the self-adapting and self-organizing function of the body, thereby turn the enemy into a friend, even a utilizable source. Co-progress means that the biological structure system obtains, through structural information, the structural characteristics from other organisms that are beneficial to health, survival and reproduction of organisms. According to the well-established theory system of *Structural information medicine*, the present disclosure relates to use of the stable structure corresponding to a substance to allow an organism to regulate its genes or gene expressions through the self-adapting and self-organizing ability of its own structure system, so as to treat diseases associated with the substance.

**Summary of Invention**

The objective of the present disclosure is to provide a method for designing a substance for treatment and/or prevention of diseases to address the above-mentioned defects in the prior art.

Another objective of the present disclosure is to provide a substances designed by the above method.

Yet another objective of the present disclosure is to provide a method for preparation and use of the substances.

The biological structure system is an organic structure system with unified stability and adaptability. It can adapt to the environment, maintain the dynamic balance between itself and the external environment, and in turn achieve better survival and reproduction for itself. The adaptability of the biological structure system is based on the stability of the structure of the organism, and the stable structure is a dominating structure that plays a key role.

The biological structure system is an organic giant system formed by spiral development of a core structure composed of concrete structures and abstract structures. The core structure of the biological structure system, that is, a unified complex of the specific structure and the abstract structure in the nucleic acid-protein structure system, is the carrier and expression system of genetic information, plays a dominating role in functioning of the biological structure system, while in the core structure it is the stable structure that plays a dominating role. At the atomic level, the abstract structure DNA and the concrete structure protein in the core structure are both biological macromolecules based on the carbon element. The outermost layer of a carbon atom has 4 electrons, and it is neither easy to lose electrons nor easy to obtain electrons. This structure favors the formation of 4 covalent bonds from a carbon atom. A covalent bond has high stability and is the strongest force between organic molecules. The single covalent bond mainly formed by carbon atoms is about 300-500 KJ/moL, and the thermal breaking temperature is 500-800°C.

The primary structure of DNA determines the double helix secondary structure of DNA through hydrogen bonding and stacking force between bases. That is, the stable primary structure determines its adaptive secondary structure, and the core of genetic information is in the stable structure. DNA transfers the genetic information to RNA through transcription, and finally the genetic information from RNA is expressed into a protein through translation. This is the law followed by all cell-based organisms. Establishment of this central law clarifies the transfer path of genetic information from DNA to RNA to protein, i.e. from the base sequence in the primary structure of DNA to the base sequence in the primary structure of RNA to the amino acid sequence in the primary structure of protein. Through base complementary pairing and triplet codons, the genetic information carrier linearly maps pieces of information one by one to the functional information carrier-amino acids. Therefore, this information transfer path is based on the stable structure of these biological macromolecules, i.e. transfer from the stable structure of the abstract structure to the stable structure of the specific structure.

The covalent backbone of the primary structure of a protein contains hundreds of single bonds which are freely rotatable, but the freedom of rotation of each single bond is restricted by factors such as the rigid planar nature of the peptide bond, the number and position of hydrophilic and hydrophobic amino acids and acidic and basic amino acids in the primary structure, finally forming the thermodynamically most stable three-dimensional structure. Therefore, in fact, the factor controlling the final structure of a protein, that is, the amino acid sequence, is formed very early. These stable structures that govern the protein conformation form a framework, according to which the polypeptide chain assembles into the final form during folding. The amino acid sequence of a protein contains all the information that determines its three-dimensional structure, and the structure of the protein determines its function. Therefore, consistent with the genetic information, the functional information also has the nature that the stable structure determines the adapted structure.

From the perspective of a system, the stability of the biological structure system is a self-stabilizing ability of the organic structure system formed via structural interactions. Each structure in the structure system is promoted by some structures and inhibited by other structures, and they form a dynamic balance via interactions that is neither deficient nor excessive and can adjust itself within a certain range according to environmental changes, to maintain and restore the original ordered state, that is, to maintain its own order from an imbalanced state, in which the interactions between the stable structures play a dominating role. For example, for the recognition of structural information, the innate immune system in animals and plants can recognize structural characteristics of pathogens through pattern recognition receptors (PRRs), and these characteristics are stable molecular structures shared by different types of pathogens and do not easily mutate (for example, all bacteria have a cell wall comprising LPS). For another example, as regards food allergies, even if food containing allergens is steamed or boiled at a high temperature, it still causes allergies as the immune recognition is directed to the stable structure of the allergens. Taking neurotransmitter opioid peptides as an example, opioid peptides in different conformations have different selectivity for cell membrane receptors. Endomorphin-1 is a biologically active peptide known to have the highest affinity and selectivity to $\mu$ receptors. An adaptive structure is the molecular basis for the binding of signal molecules to receptors. From the perspective of variations in the stable structure amino acids, endomorphin-1 differs from other opioid peptides mainly in three amino acids. By the change in amino acid residues, it forms an appropriate spatial layout, changes its three-dimensional conformation, and provides correct positioning for binding of such transmitters to receptors. Interactions between the stable structures can cause significant changes in the adaptive structures, and play a crucial role in information recognition. During information transfer, receptors on the cell membrane need to bring the information to the nucleus, which mainly relies on modifications of proteins. The basis of protein modification is the chemical reactivity of amino acid residues in the primary structure. Under the action of enzymes, the stable structures of a protein can be adjusted to make the higher structure of the protein more adapted to the functional requirements under specific time and spatial conditions, with reversibility and versatility, for example phosphorylation and de-phosphorylation of proteins. To the information transferred to the nucleus, the cell eventually makes a response by altering the gene expression pattern, and in turn passes instructions down by signaling pathways. Therefore, interactions between the stable structures or adjustment of the structures determine the changes in the adaptive structures, and certainly the adaptive structures can also make a reaction on the stable structures.

In the biological structure system, no structure is "isolated". The interactions, mutual restriction and interdependence between structures and between structural sub-systems constitute the organic biological structure system. All organisms are made of cells, and cells are the structural sub-systems of the large biological structure system. Depending on its complexity, an organism may be composed of hundreds, tens of thousands, or even hundreds of millions of cells that exert various specific functions. The cells divide the work and cooperate to build an ordered and controllable cell society. Maintenance of such a society depends not only on the material metabolism and energy metabolism of the cells, but also on the communication and signal regulation between the cells, so that the behaviors of the cells, such as cell growth, division, differentiation, apoptosis and other physiological functions, can be coordinated to realize a complete life activity process of a multicellular organism. The essence of intercellular communication and signal regulation is also interaction between structures which determine the behavior and fate of cells, including structural and functional differentiation, location, and fateful choices. The morphology and structure, life activity and position of cells in the body are all regulated and controlled by the body, local tissue, surrounding cells, and extracellular signaling molecules. For example, nerve cells, immune cells and endocrine cells are in social communication to participate in and maintain the homeostasis of the body. The interaction between structures in the biological structure system makes the cell capable of distinguishing itself from others, and distinguishing between allogenic cells and heterogenic homologous cells. The phenomenon of mutual identification and recognition among cells, as well as the molecular recognition of self and foreign substances, is called cell recognition. Based on the recognition, cells adhere to each other or establish a stable connection with a certain morphology and structure, thereby influencing and interacting with each other. Many important life activities are associated with the recognition ability of cells. Cell recognition is also a very important step in the process of cell development and differentiation. Cells form different types of tissue through recognition and adhesion. Body immunity is also in fact a phenomenon of cell recognition. White blood cells in the blood can recognize invading bacteria and engulf them, but never engulf own normal cells in the blood. This is cell recognition between different species. After clinic transplantation of allogeneic tissue, the body will reject the transplanted allogeneic tissue, which is caused by the cell recognition between allogeneic cells. In addition, many physiological and pathological processes such as blood coagulation, inflammatory reaction, thrombosis, infections by pathogenic microorganisms and even tumor cells metastasis, are associated with the complex mutual recognition and adhesion between the allogenic and the xenogenic as well as the effects induced thereby. Structure recognition is based on the interaction between structures, which the basis of the self-adaptive, self-organizing organic structure system formed in an organism.

The openness of a structure system is not only the openness of specific structures, but also the openness of relationships. The self-adapting and self-organizing ability of the structure system is mainly reflected in the stabilization of the system under external environmental stimuli. The biological structure system makes self-adjustments and self-organizations through transmission and processing of information, and constantly overcomes the uncertainty in the structure system to keep it in a dynamic balance. The exchange between the structure system and its environment is not only exchange of substance, but also exchange of energy or information. To maintain the homeostasis in a constantly changing environment, an organism needs the ability of cells to sense and respond to these changes. The ability of cells to receive and transmit information and execute instructions is the basis for the organism to respond to changes in internal and external environments. Proteins are the performers of various physiological activities in a cell (such as catalyzing chemical reactions and regulating gene expressions), and changes in the own state of proteins will also change their functioning. Therefore organisms use protein molecules possessed by cells to build a signaling system. Because the shape of a protein molecule can be changed, the functions closely related to the shape of the protein molecule will also change accordingly, and one change in the state of the protein will change the state of the downstream molecules through interaction with downstream protein molecules, which in turn further change the state of more downstream molecules. Such a cascade of changes in state is the way information is transmitted in a cell. Moreover, proteins are biologically functional molecules that make a final response to the signal after the change in state, and become effectors. The effectors at the end of the information transmission chain are mostly transcription factors, which respond to signals by regulating expressions of genes.

Taking the immune system as an example, any external structure having entered the human body will be recognized and evaluated by the immune system. For example, when bacteria or viruses enter the human body, immune cells interact with the foreign structures to recognize their structural information, and then secrete different cytokines according to the different structural information and transmit the information to B lymphocytes and regulate the gene expression of B cells to synthesize IgG antibodies that bind the antigen structure to form a complex, which is eventually eliminated by macrophages to resist foreign invasion. When parasitic pathogens invade, immune cells interact with the foreign structure to recognize structural information that activates B cells and regulates gene expression to synthesize IgE antibodies. These antibodies bind mast cells and basophils to release toxic granule proteins and the like from the cells to kill the parasites.

In this immune response, the recognized structure determines the specific feedback of the body. Through the interaction between structures, specific structures turn into an abstract structure-structural information. Based on the recognition of the structural information, the body mobilizes multiple cells in the immune system to work coordinately, regulates gene expressions, and releases different active substances to adaptively act against foreign objects and achieve a new stable and harmonious structure system. Meanwhile the immune system interacts with other systems to exchange material, energy, and information, to realize coordination of different sub-systems in the structure system. It can be seen that there are structural information recognition and self-regulation in the biological structure system, which restore the balance of the structure system, and even establish a new structural balance mechanism with external structures.

A disease is an abnormal life process that occurs due to a disorder of homeostasis under the action of a certain cause, and triggers a series of changes in metabolism, function, and structure, manifested in symptoms, signs, and abnormal behaviors. A disease is an abnormal life process that occurs due to a disorder of homeostasis after the body is damaged by a cause of disease under certain conditions. From a structural point of view, diseases of an organism are caused by imbalance of the biological structure system, including: (1) structural imbalance within the biological structure system; and (2) imbalance between the biological structure system and an external structure, that is, a substance inhibiting the external structure is absent in the biological structure system. Therefore, substances associated with diseases include: a substance that causes diseases, an organism's own substance associated with diseases, and a substance that inhibits the structures that cause diseases. The substance that inhibits the structures that cause diseases is a substance that can establish a structural balance with the structures that cause diseases. The disease is an extremely complex process, and in many cases, turning from health to disease is a qualitative change from quantitative changes. Some diseases involve structural imbalance of multiple systems in the biological structure system. The term "*indication*" used in traditional Chinese medicine reflects the essence of diseases, and the "*indication*" is identified to find a therapy (i.e. identification of indication then treatment). The "*indication*" is neither a disease/disorder described in Western medicine, nor a generalization of a single factor or linear causality of a disease. It is an interpretation and generalization of the responses of the body in connection with various physiological and pathological events at a certain stage of a disease, such as the cause of disease, site of disease, pathogenesis, pathological nature, and the combat between *the positive* and *the negative*, based on the unique theories and methodology of traditional Chinese medicine. It focuses on the unification of *the positive* and *the negative* of the body which are opposite to each other. Therefore, the fundamental principle of therapy in traditional Chinese medicine is to "*support the positive to eliminate the negative*", and "*eliminate the negative to secure the positive*", thereby adjusting the *Yin and Yang* in the human body based on both "*the positive*" and "*the negative*"*,* restoring a balance therebetween, and coordinating the ordered homeostasis of "*the positive*" and "*the negative*".

Western medicine studies the structure-function relationship from a concrete, microscopic perspective, so as to study the health and disease. Traditional Chinese medicine studies the multidimensional mutual-regulating relationships (such as promoting, inhibiting, restricting, and assimilating) between the human body's functional systems from an abstract, macroscopic perspective, so as to study health and diseases. From a structural point of view, the essence of an organism is a dynamically balanced, coordinated, and ordered self-adaptive, self-organizing organic structural system formed by interactions between body structures. *Structural information medicine* integrates the basic theories of traditional Chinese medicine and Western medicine with structures, interprets life from the perspective of the structure system, and restores the dynamic balance of the structure system by allowing the body to recognize the information of a specific structure and use the self-adapting and self-organizing function of the body's structure system to achieve the purpose of treating and preventing diseases.

Different pathological changes in the same organ may cause different diseases, the symptoms of the same disease in different individuals are not exactly the same, and the same disease may involve pathological changes in multiple different organs or tissues in the biological structure system, and the pathologically changed structures in the same disease are not exactly the same in different individuals. Therefore, the present disclosure combines the specific structure studied by Western medicine and the macroscopic theory established by traditional Chinese medicine to treat and/or prevent diseases from the perspective of structural information. For a disease in an organism, only the substances associated with the disease, as well as the specific pathologically changed cells, tissue or organs where the substances associated with the disease are present, are needed to know; and there is no need to investigate the mechanism of pathogenicity, specific symptoms of the disease, or the system where the substances associated with the disease are present, especially when the specific pathologically changed structure cannot be determined, or it is not easy to isolate the structure. Humans are the same as each other from a systemic point of view, according to both Western medicine and traditional Chinese medicine, and humans are even substantially similar to swine, bovine, Caprinae, and primate, so it is more versatile to use and also easier to obtain and prepare the stable structure corresponding to the cells, tissue or organs containing the pathologically changed structures (that is, the imbalanced structures) for treatment and/or prevention of diseases. Therefore, the method for designing a substance for treatment and/or prevention of diseases according to the present disclosure uses the stable structure corresponding to a substance associated with disease as an external structure to act on the biological structure system, so that the biological structure system can use its self-adaption and self-organization according to the structure to establish a balance with the structure of the external structure or restore the internal balance of the biological structure system, thereby eliminating, alleviating, or preventing the diseases. For example, the stable structure corresponding to the lung of swine is used to treat and/or prevent asthma, or allergic rhinitis which is a disease in the nose in interaction with the structure of the lung, i.e. a disease associated with the lung. The method for designing a substance for treatment and/or prevention of diseases according to the present disclosure mainly includes supporting "*the positive*" by "*the negative*" and supporting "*the positive*" by "*the positive*".

The imbalance of the biological structure system includes the imbalance between the biological structure system itself and a non-self-structure. In the present disclosure, the biological structure system itself is called "*the positive (ZHENG, or GOOD)*", and the external structure that causes imbalance of the biological structure system is called "*the external negative (external XIE, or external BAD)*". "*The external negative*" is with respect to an organism, including substances harmful to a healthy organism, such as pathogenic bacteria and viruses, and also including substances that are harmless to a healthy organism, but in certain organisms induce onset of diseases upon contact, despite that there is no onset of diseases in the absence of the substances. For example, some substances are harmless or even beneficial to healthy organisms, indicating that the structure of these substances is in a balance with the structure of the organisms. However, if the structure of the organism is imbalanced, the structural balance between the substances and the organisms is broken, and various imbalances may cause different diseases, such as allergies or intolerance to these substances, etc., such as milk allergy and milk intolerance. Under the action of an external structure, with the self-adapting and self-organizing function of the biological structure system that maintains the structural stability, a structure that interacts with the external structure in an inhibitory or promoting manner will be generated, thereby establishing a balance between the biological structure system and the external structure, i.e. supporting the *"positive"* by the *"external negative"*.

A pathological change in the internal structure of the biological structure system is often imbalance of structural interaction. For example, for a certain structure in the biological structure system, under-expression of genes of structures restricting it or over-expression of genes of structures promoting it will break the balance between structures. For example, tumor suppressor genes and proto-oncogenes restrict each other to maintain relative stability of positive and negative regulatory signals. When such genes are mutated, deleted or inactivated, the balance of interactions between structures is broken, which may cause malignant transformation of cells and lead to tumors. The present disclosure refers to the imbalanced structure in the biological structure system as "*the internal negative (internal XIE)*", and refers to the biological structure system as *"the positive"*. According to the present disclosure, the stable structure corresponding to the imbalanced internal structure of an organism is used as an external structure to act on the biological structure system. Due to the self-adapting, self-organizing and self-stabilizing function of the biological structure system, the biological structure system will regulate the expression of genes of structures that interact with the external structure according to the structural information from the external structure, so as to achieve a balance with the external structure, thus realizing restoration of an internal balance for the imbalanced structure in the biological structure system and treatment of diseases, i.e. supporting the "*positive*" by the "*internal negative*".

For the imbalanced internal structures in the biological structure system, its corresponding balanced structures in each case has a structure interacting with it; and these balanced structures are also referred to as "*the positive*" in the present disclosure.

Each normal structure in the biological structure system is interacting with some other structures, i.e. balanced structures, and the present disclosure refers to both the balanced structures and the biological structure system as "*the positive*". The stable structure corresponding to a normal, pathologically unchanged structure in the structure system is used as an external structure to act on the biological structure system. Due to the self-adapting, self-organizing and self-stabilizing function of the biological structure system, the biological structure system will regulate the expression of genes of structures that interact with the external structure to a normal level according to the structural information from each structure in the external structure, so that the imbalanced structure restores the balance. According to the present disclosure, the stable structure corresponding to a pathologically unchanged substance is used as a substance for treatment and/or prevention of diseases caused by a pathological change in a substance identical or similar to the pathologically unchanged substance, i.e. supporting the "*positive*" by the "*positive*".

The balance of the biological structure system is dynamic, especially the balance between the biological structure system and the structure of the environment surrounding the organism. For example, a substance capable of causing a disease may not cause the disease in a certain organism, in which case the structure system of the certain organism can achieve a balance with the structure of the disease-causing substance upon interaction, that is to say, the disease-associated structure in the certain organism has a structure that interacts with the structure of the disease-causing substance and prevents the disease in the organism, and such a structure is not isolated but in interactions with other structures in the biological structure system, thereby reaching a balance. The stable structure corresponding to the disease-associated structure in a certain organism is used as an external structure to act on the biological structure system in need of treatment of this disease. Due to the self-adapting, self-organizing and self-stabilizing function of the biological structure system, the biological structure system will regulate the genes or expression of genes of structures that interact with the external structure according to the structural information from each structure in the external structure, so that a balance is achieved between the biological structure system and the structure of the disease-causing substance, which is also supporting the "*positive*" by the "*positive*".

Features of the present disclosure:

According to the well-established theory system of *Structural information medicine*, the present disclosure uses a stable structure corresponding to a substance to act on the biological structure system, and allows the organism to regulate its genes or gene expressions through the self-adapting and self-organizing ability of its own structure system, so as to treat diseases associated with the substance.

In view of this, the present disclosure provides a substance for treatment and/or prevention of diseases, the method for designing the substance, and the method for preparing the substance. The specific embodiments thereof are as follows.

A method for designing a substance for treatment and/or prevention of diseases, comprising:
using a stable structure corresponding to a substance associated with disease as the substance for treatment and/or prevention of the diseases.

Preferably, the stable structure corresponding to the substance refers to the structure of the substance that is present in a solid state at a high temperature of 500°C or more.

Preferably, the stable structure corresponding to a substance associated with disease is one or more selected from:

(1) a stable structure corresponding to a substance causing the disease in an organism;
(2) a stable structure corresponding to a substance associated with the disease from an organism itself;
(3) a stable structure corresponding to a substance containing a disease-inhibiting structure;
(4) a stable structure corresponding to a substance containing the substance according to any one of (1) to (3) above.

A substance for treatment and/or prevention of diseases, designed according to the above method.

A method for preparing the above substance for treatment and/or prevention of diseases, wherein the stable structure corresponding to the substance is prepared by high-temperature carbonization.

Use of the above substance for treatment and/or prevention of diseases in the manufacture of medicaments, health products, food, and food additives.

A method for designing a substance for treatment and/or prevention of depression, comprising:
using a stable structure corresponding to a depression-associated substance as the substance for treatment and/or prevention of depression.

Preferably, the stable structure corresponding to the substance refers to the structure of the substance that is present in a solid state at a high temperature of 500°C or more.

Depression is a chronic recurrent disease having both affective and physical symptoms.

The neurotransmitter serotonin (5-HT) in patients' brain is disordered or deficient, resulting in significant and lasting depressed mood. Depression-associated pathologically changed substances are mainly involved in the nervous system and endocrine system.

Preferably, the stable structure corresponding to a depression -associated substance is one or more selected from:

(1) a stable structure corresponding to a substance causing depression in an organism;
(2) a stable structure corresponding to a substance of an organism itself associated with depression;
(3) a stable structure corresponding to a substance containing a depression-inhibiting structure;
(4) a stable structure corresponding to a substance containing the substance according to any one of (1) to (3) above.

In another preferred embodiment, the depression-associated substance is selected from one or more of substances in the nervous system, and substances in the endocrine system.

A substance for treatment and/or prevention of depression, designed according to the above method.

A method for preparing the above substance for treatment and/or prevention of depression, wherein the stable structure corresponding to the substance is prepared by high-temperature carbonization.

The content (substance) in the cranial cavity of an organism contain depression-associated pathologically changed substances, and are easy to obtain. Therefore, it is preferable to prepare the substance for treatment and/or prevention of depression by high-temperature carbonization of the content in the cranial cavity of an organism. If other organs or tissue, such as the adrenal glands, also contains depression-associated pathologically changed substances, it is also preferable to use these depression-associated pathologically changed substances to prepare the substance for treatment and/or prevention of depression by high-temperature carbonization. In doing so, complex problems are simplified, and substances for treatment and/or prevention of depression can be designed and prepared without knowing the specific pathologically changed structure or the mechanism of pathogenicity. In the present disclosure, it is preferable to use content in the cranial cavity of Caprinae to prepare the substance for treatment and/or prevention of depression by high-temperature carbonization.

Use of the above substance for treatment and/or prevention of depression in the manufacture of medicaments, health products, food, and food additives.

A method for designing a substance for treatment and/or prevention of anxiety, comprising:
using a stable structure corresponding to an anxiety-associated substance as the substance for treatment and/or prevention of anxiety.

Preferably, the stable structure corresponding to the substance refers to the structure of the substance that is present in a solid state at a high temperature of 500°C or more.

Preferably, the stable structure corresponding to an anxiety-associated substance is one or more selected from:

(1) a stable structure corresponding to a substance causing anxiety in an organism;
(2) a stable structure corresponding to a substance of an organism itself associated with anxiety;

(3) a stable structure corresponding to a substance containing an anxiety-inhibiting structure;

(4) a stable structure corresponding to a substance containing the substance according to any one of (1) to (3) above.

In another preferred embodiment, the anxiety-associated substance is selected from one or more of substances in the nervous system and substances in the endocrine system.

A substance for treatment and/or prevention of anxiety, designed according to the above method.

A method for preparing the above substance for treatment and/or prevention of anxiety, wherein the stable structure corresponding to the substance is prepared by high-temperature carbonization.

The content in the cranial cavity of an organism contain anxiety-associated pathologically changed substances, and are easy to obtain. Therefore, it is preferable to prepare the substance for treatment and/or prevention of anxiety by high-temperature carbonization of the content in the cranial cavity of an organism. If other organs or tissue also contains anxiety-associated pathologically changed substances, it is also preferable to use these anxiety-associated pathologically changed substances to prepare the substance for treatment and/or prevention of anxiety by high-temperature carbonization. In doing so, complex problems are simplified, and substances for treatment and/or prevention of anxiety can be designed and prepared without knowing the specific pathologically changed structure or the mechanism of pathogenicity. In the present disclosure, it is preferable to use content in the cranial cavity of Caprinae to prepare the substance for treatment and/or prevention of anxiety by high-temperature carbonization.

Use of the above substance for treatment and/or prevention of anxiety in the manufacture of medicaments, health products, food, and food additives.

A method for designing a substance for treatment and/or prevention of mania, comprising:

using a stable structure corresponding to a mania-associated substance as the substance for treatment and/or prevention of mania.

Preferably, the stable structure corresponding to the substance refers to the structure of the substance that is present in a solid state at a high temperature of 500°C or more.

Preferably, the stable structure corresponding to a mania-associated substance is one or more selected from:

(1) a stable structure corresponding to a substance causing mania in an organism;

(2) a stable structure corresponding to a substance of an organism itself associated with mania;

(3) a stable structure corresponding to a substance containing a mania-inhibiting structure;

(4) a stable structure corresponding to a substance containing the substance according to any one of (1) to (3) above.

In another preferred embodiment, the mania-associated substance is selected from one or more of substances in the nervous system and substances in the endocrine system.

A substance for treatment and/or prevention of mania, designed according to the above method.

A method for preparing the above substance for treatment and/or prevention of mania, wherein the stable structure corresponding to the substance is prepared by high-temperature carbonization.

The content in the cranial cavity of an organism contain mania-associated pathologically changed substances, and are easy to obtain. Therefore, it is preferable to prepare the substance for treatment and/or prevention of mania by high-temperature carbonization of the content in the cranial cavity of an organism. If other organs or tissue also contains mania-associated pathologically changed substances, it is also preferable to use these mania-associated pathologically changed substances to prepare the substance for treatment and/or prevention of mania by high-temperature carbonization. In doing so, complex problems are simplified, and substances for treatment and/or prevention of mania can be designed and prepared without knowing the specific pathologically changed structure or the mechanism of pathogenicity. In the present disclosure, it is preferable to use content in the cranial cavity of Caprinae to prepare the substance for treatment and/or prevention of mania by high-temperature carbonization.

Use of the above substance for treatment and/or prevention of mania in the manufacture of medicaments, health products, food, and food additives.

A method for designing a substance for treatment and/or prevention of bulimia, comprising:

using a stable structure corresponding to a bulimia-associated substance as the substance for treatment and/or prevention of bulimia.

Preferably, the stable structure corresponding to the substance refers to the structure of the substance that is present in a solid state at a high temperature of 500°C or more.

Preferably, the stable structure corresponding to a bulimia-associated substance is one or more selected from:

(1) a stable structure corresponding to a substance causing bulimia in an organism;

(2) a stable structure corresponding to a substance of an organism itself associated with bulimia;

(3) a stable structure corresponding to a substance containing a bulimia-inhibiting structure;

(4) a stable structure corresponding to a substance containing the substance according to any one of (1) to (3) above.

In another preferred embodiment, the bulimia-associated substance is selected from one or more of substances in the nervous system and substances in the endocrine system.

A substance for treatment and/or prevention of bulimia, designed according to the above method.

A method for preparing the above substance for treatment and/or prevention of bulimia, wherein the stable structure corresponding to the substance is prepared by high-temperature carbonization.

The content in the cranial cavity of an organism contain bulimia-associated pathologically changed substances, and are easy to obtain. Therefore, it is preferable to prepare the substance for treatment and/or prevention of bulimia by high-temperature carbonization of the content in the cranial cavity of an organism. If other organs or tissue also contains bulimia-associated pathologically changed substances, it is also preferable to use these bulimia-associated pathologically changed substances to prepare the substance for treatment and/or prevention of bulimia by high-temperature carbonization. In doing so, complex problems are simplified, and substances for treatment and/or prevention of bulimia can be designed and prepared without knowing the specific pathologically changed structure or the mechanism of pathogenicity. In the present disclosure, it is preferable to use content in the cranial cavity of Caprinae to prepare the substance for treatment and/or prevention of bulimia by high-temperature carbonization.

Use of the above substance for treatment and/or prevention of bulimia in the manufacture of medicaments, health products, food, and food additives.

A method for designing a substance for treatment and/or prevention of anorexia, comprising:

using a stable structure corresponding to an anorexia-associated substance as the substance for treatment and/or prevention of anorexia.

Preferably, the stable structure corresponding to the substance refers to the structure of the substance that is present in a solid state at a high temperature of 500°C or more.

Preferably, the stable structure corresponding to an anorexia-associated substance is one or more selected from:

(1) a stable structure corresponding to a substance causing anorexia in an organism;
(2) a stable structure corresponding to a substance of an organism itself associated with anorexia;
(3) a stable structure corresponding to a substance containing an anorexia-inhibiting structure;
(4) a stable structure corresponding to a substance containing the substance according to any one of (1) to (3) above.

In another preferred embodiment, the anorexia-associated substance is selected from one or more of substances in the nervous system, substances in the endocrine system, the ovary, and the intestinal tract of an organism.

A substance for treatment and/or prevention of anorexia, designed according to the above method.

A method for preparing the above substance for treatment and/or prevention of anorexia, wherein the stable structure corresponding to the substance is prepared by high-temperature carbonization.

Use of the above substance for treatment and/or prevention of anorexia in the manufacture of medicaments, health products, food, and food additives.

A method for designing a substance for treatment and/or prevention of insomnia, comprising:

using a stable structure corresponding to an insomnia-associated substance as the substance for treatment and/or prevention of insomnia.

Preferably, the stable structure corresponding to the substance refers to the structure of the substance that is present in a solid state at a high temperature of 500°C or more.

Preferably, the stable structure corresponding to an insomnia-associated substance is one or more selected from:

(1) a stable structure corresponding to a substance causing insomnia in an organism;
(2) a stable structure corresponding to a substance of an organism itself associated with insomnia;
(3) a stable structure corresponding to a substance containing an insomnia-inhibiting structure;
(4) a stable structure corresponding to a substance containing the substance according to any one of (1) to (3) above.

In another preferred embodiment, the insomnia-associated substance is selected from one or more of substances in the nervous system, and substances in the endocrine system.

A substance for treatment and/or prevention of insomnia, designed according to the above method.

A method for preparing the above substance for treatment and/or prevention of insomnia, wherein the stable structure corresponding to the substance is prepared by high-temperature carbonization.

Use of the above substance for treatment and/or prevention of insomnia in the manufacture of medicaments, health products, food, and food additives.

A method for designing a substance for treatment and/or prevention of tension headache, comprising:

using a stable structure corresponding to a tension headache-associated substance as the substance for treatment and/or prevention of tension headache.

Preferably, the stable structure corresponding to the substance refers to the structure of the substance that is present in a solid state at a high temperature of 500°C or more.

Preferably, the stable structure corresponding to a tension headache-associated substance is one or more selected from:

(1) a stable structure corresponding to a substance causing tension headache in an organism;
(2) a stable structure corresponding to a substance of an organism itself associated with tension headache;
(3) a stable structure corresponding to a substance containing a tension headache-inhibiting structure;
(4) a stable structure corresponding to a substance containing the substance according to any one of (1) to (3) above.

In another preferred embodiment, the tension headache-associated substance is selected from one or more of substances in the nervous system and substances in the endocrine system.

A substance for treatment and/or prevention of tension headache, designed according to the above method.

A method for preparing the above substance for treatment and/or prevention of tension headache, wherein the stable structure corresponding to the substance is prepared by high-temperature carbonization.

The content in the cranial cavity of an organism contain tension headache-associated pathologically changed substances, and are easy to obtain. Therefore, it is preferable to prepare the substance for treatment and/or prevention of tension headache by high-temperature carbonization of the content in the cranial cavity of an organism. If other organs or tissue also contains tension headache-associated pathologically changed substances, it is also preferable to use these tension headache-associated pathologically changed substances to prepare the substance for treatment and/or prevention of tension headache by high-temperature carbonization. In doing so, complex problems are simplified, and substances for treatment and/or prevention of tension headache can be designed and prepared without knowing the specific pathologically changed structure or the mechanism of pathogenicity. In the present disclosure, it is preferable to use content in the cranial cavity of Caprinae to prepare the substance for treatment and/or prevention of tension headache by high-temperature carbonization.

Use of the above substance for treatment and/or prevention of tension headache in the manufacture of medicaments, health products, food, and food additives.

A method for designing a substance for treatment and/or prevention of diabetes, comprising:
using a stable structure corresponding to a diabetes-associated substance as the substance for treatment and/or prevention of diabetes.

Preferably, the stable structure corresponding to the substance refers to the structure of the substance that is present in a solid state at a high temperature of 500°C or more.

Preferably, the stable structure corresponding to a diabetes-associated substance is one or more selected from:

(1) a stable structure corresponding to a substance causing diabetes in an organism;
(2) a stable structure corresponding to a substance of an organism itself associated with diabetes;
(3) a stable structure corresponding to a substance containing a diabetes-inhibiting structure;
(4) a stable structure corresponding to a substance containing the substance according to any one of (1) to (3) above.

In another preferred embodiment, the diabetes-associated substance is derived from a secreting gland of an organism, preferably the islets of pancreas and/or adrenal glands of an organism.

Diabetes is a syndrome of a series of metabolic disorders of proteins, fats, electrolytes, and the like caused by absolute or relative insufficient secretion of insulin and decreased sensitivity of target tissue cells to insulin. A variety of hormones secreted by the adrenal medulla affect glucose metabolism, antagonize the effect of insulin, and increase the blood sugar level. Diabetes-associated pathologically changed substances are mainly involved in the islets of pancreas and/or adrenal glands of an organism.

A substance for treatment and/or prevention of diabetes, designed according to the above method.

A method for preparing the above substance for treatment and/or prevention of diabetes, wherein the stable structure corresponding to the substance is prepared by high-temperature carbonization.

The substances in the islets of pancreas and/or adrenal glands of an organism contain diabetes-associated pathologically changed substances, and are easy to obtain. Therefore, it is preferable to prepare the substance for treatment and/or prevention of diabetes by high-temperature carbonization of the substances in the islets of pancreas and/or adrenal glands of an organism. If other organs or tissue also contains diabetes-associated pathologically changed substances, it is also preferable to use these diabetes-associated pathologically changed organs or tissue to prepare the substance for treatment and/or prevention of diabetes by high-temperature carbonization. In doing so, complex problems are simplified, and substances for treatment and/or prevention of diabetes can be designed and prepared without knowing the specific pathologically changed structure or the mechanism of pathogenicity. In the present disclosure, it is preferable to use substances in the islets of pancreas and/or adrenal glands of swine to prepare the substance for treatment and/or prevention of diabetes by high-temperature carbonization.

Use of the above substance for treatment and/or prevention of diabetes in the manufacture of medicaments, health products, food, and food additives.

A method for designing a substance for treatment and/or prevention of hypertension, comprising: using a stable structure corresponding to a hypertension-associated substance as the substance for treatment and/or prevention of hypertension.

Preferably, the stable structure corresponding to the substance refers to the structure of the substance that is present in a solid state at a high temperature of 500°C or more.

Preferably, the stable structure corresponding to a hypertension-associated substance is one or more selected from:

(1) a stable structure corresponding to a substance causing hypertension in an organism;
(2) a stable structure corresponding to a substance of an organism itself associated with hypertension;
(3) a stable structure corresponding to a substance containing a hypertension-inhibiting structure;
(4) a stable structure corresponding to a substance containing the substance according to any one of (1) to (3) above.

In another preferred embodiment, the hypertension-associated substance is selected from one or more of substances in the nervous system, substances in the endocrine system, viscera, and blood vessels of an organism.

Most hypertensive patients, especially in the early stage of hypertension, show signs of increased sympathetic nerve activity: the release of catecholamines, epinephrine, and norepinephrine in the adrenal medulla increases, and the level of adrenaline in the blood continues to increase. Catecholamines act on the central nervous system, increase its excitability, enhance the release of norepinephrine from sympathetic nerve endings, increase cardiac output, and elevate the blood pressure. Therefore, hypertension-associated pathologically changed substances are involved in the nervous system, endocrine system, such as the adrenal glands, and kidneys.

A substance for treatment and/or prevention of hypertension, designed according to the above method.

A method for preparing the above substance for treatment and/or prevention of hypertension, wherein the stable structure corresponding to the substance is prepared by high-temperature carbonization.

The content in the cranial cavity and the adrenal glands of an organism contain hypertension-associated pathologically changed substances, and are easy to obtain. Therefore, it is preferable to prepare the substance for treatment and/or prevention of hypertension by high-temperature carbonization of the content in the cranial cavity of an organism and/or the adrenal glands. If other organs or tissue also contains hypertension-associated pathologically changed substances, it is also preferable to use these hypertension-associated pathologically changed organ or tissue to prepare the substance for treatment and/or prevention of hypertension by high-temperature carbonization. In doing so, complex problems are simplified, and substances for treatment and/or prevention of hypertension can be designed and prepared without knowing the specific pathologically changed structure or the mechanism of pathogenicity. In the present disclosure, it is preferable to use content in the cranial cavity of Caprinae and/or the adrenal glands of swine to prepare the substance for treatment and/or prevention of hypertension by high-temperature carbonization.

Use of the above substance for treatment and/or prevention of hypertension in the manufacture of medicaments, health products, food, and food additives.

A method for designing a substance for treatment and/or prevention of menoxenia, comprising: using a stable structure corresponding to a menoxenia-associated substance as the substance for treatment and/or prevention of menoxenia.

Preferably, the stable structure corresponding to the substance refers to the structure of the substance that is present in a solid state at a high temperature of 500°C or more.

Preferably, the stable structure corresponding to a menoxenia-associated substance is one or more selected from:

(1) a stable structure corresponding to a substance causing menoxenia in an organism;
(2) a stable structure corresponding to a substance of an organism itself associated with menoxenia;
(3) a stable structure corresponding to a substance containing a menoxenia-inhibiting structure;
(4) a stable structure corresponding to a substance containing the substance according to any one of (1) to (3) above.

In another preferred embodiment, the menoxenia-associated substance is selected from one or more of substances in the nervous system, substances in the endocrine system, the ovary, and the spleen.

A substance for treatment and/or prevention of menoxenia, designed according to the above method.

A method for preparing the above substance for treatment and/or prevention of menoxenia, wherein the stable structure corresponding to the substance is prepared by high-temperature carbonization.

The content in the cranial cavity of an organism contain menoxenia-associated pathologically changed substances, and are easy to obtain. Therefore, it is preferable to prepare the substance for treatment and/or prevention of menoxenia by high-temperature carbonization of the content in the cranial cavity of an organism. If other organs or tissue, such as the ovary or spleen, also contains menoxenia-associated pathologically changed substances, it is also preferable to use these menoxenia-associated pathologically changed organs or tissue to prepare the substance for treatment and/or prevention of menoxenia by high-temperature carbonization. In doing so, complex problems are simplified, and substances for treatment and/or prevention of menoxenia can be designed and prepared without knowing the specific pathologically changed structure or the mechanism of pathogenicity. In the present disclosure, it is preferable to use content in the cranial cavity of Caprinae to prepare the substance for treatment and/or prevention of menoxenia by high-temperature carbonization.

Use of the above substance for treatment and/or prevention of menoxenia in the manufacture of medicaments, health products, food, and food additives.

A method for designing a substance for treatment and/or prevention of amenorrhea, comprising:
using a stable structure corresponding to an amenorrhea-associated substance as the substance for treatment and/or prevention of amenorrhea.

Preferably, the stable structure corresponding to the substance refers to the structure of the substance that is present in a solid state at a high temperature of 500°C or more.

Preferably, the stable structure corresponding to an amenorrhea-associated substance is one or more selected from:

(1) a stable structure corresponding to a substance causing amenorrhea in an organism;
(2) a stable structure corresponding to a substance of an organism itself associated with amenorrhea;
(3) a stable structure corresponding to a substance containing an amenorrhea-inhibiting structure;
(4) a stable structure corresponding to a substance containing the substance according to any one of (1) to (3) above.

In another preferred embodiment, the amenorrhea-associated substance is selected from one or more of substances in the nervous system, substances in the endocrine system, and the ovary.

A substance for treatment and/or prevention of amenorrhea, designed according to the above method.

A method for preparing the above substance for treatment and/or prevention of amenorrhea, wherein the stable structure corresponding to the substance is prepared by high-temperature carbonization.

The content in the cranial cavity of an organism contain amenorrhea-associated pathologically changed substances, and are easy to obtain. Therefore, it is preferable to prepare the substance for treatment and/or prevention of amenorrhea by high-temperature carbonization of the content in the cranial cavity of an organism. If other organs or tissue, such as the ovary, also contains amenorrhea-associated pathologically changed substances, it is also preferable to use these amenorrhea-associated pathologically changed organs or tissue to prepare the substance for treatment and/or prevention of amenorrhea by high-temperature carbonization. In doing so, complex problems are simplified, and substances for treatment and/or prevention of amenorrhea can be designed and prepared without knowing the specific pathologically changed structure or the mechanism of pathogenicity. In the present disclosure, it is preferable to use content in the cranial cavity of Caprinae to prepare the substance for treatment and/or prevention of amenorrhea by high-temperature carbonization.

Use of the above substance for treatment and/or prevention of amenorrhea in the manufacture of medicaments, health products, food, and food additives.

A method for designing a substance for treatment and/or prevention of dysmenorrhea, comprising:
using a stable structure corresponding to a dysmenorrhea-associated substance as the substance for treatment and/or prevention of dysmenorrhea.

Preferably, the stable structure corresponding to the substance refers to the structure of the substance that is present in a solid state at a high temperature of 500°C or more.

Preferably, the stable structure corresponding to a dysmenorrhea-associated substance is one or more selected from:

(1) a stable structure corresponding to a substance causing dysmenorrhea in an organism;
(2) a stable structure corresponding to a substance of an organism itself associated with dysmenorrhea;
(3) a stable structure corresponding to a substance containing a dysmenorrhea-inhibiting structure;
(4) a stable structure corresponding to a substance containing the substance according to any one of (1) to (3) above.

In another preferred embodiment, the dysmenorrhea-associated substance is selected from one or more of substances in the nervous system, substances in the endocrine system, and the ovary.

A substance for treatment and/or prevention of dysmenorrhea, designed according to the above method.

A method for preparing the above substance for treatment and/or prevention of dysmenorrhea, wherein the stable structure corresponding to the substance is prepared by high-temperature carbonization.

The content in the cranial cavity of an organism contain dysmenorrhea-associated pathologically changed substances, and are easy to obtain. Therefore, it is preferable to prepare the substance for treatment and/or prevention of dysmenorrhea by high-temperature carbonization of the content in the cranial cavity of an organism. If other organs or tissue also contains dysmenorrhea-associated pathologically changed substances, it is also preferable to use these dysmenorrhea-associated pathologically changed organs or tissue to prepare the substance for treatment and/or prevention of dysmenorrhea by high-temperature carbonization. In doing so, complex problems are simplified, and substances for treatment and/or prevention of dysmenorrhea can be designed and prepared without knowing the specific pathologically changed structure or the mechanism of pathogenicity. In the present disclosure, it is preferable to use content in the cranial cavity of Caprinae to prepare the substance for treatment and/or prevention of dysmenorrhea by high-temperature carbonization.

Use of the above substance for treatment and/or prevention of dysmenorrhea in the manufacture of medicaments, health products, food, and food additives.

A method for designing a substance for treatment and/or prevention of stroke sequelae, comprising:
using a stable structure corresponding to a stroke sequelae-associated substance as the substance for treatment and/or prevention of stroke sequelae.

Preferably, the stable structure corresponding to the substance refers to the structure of the substance that is present in a solid state at a high temperature of 500°C or more.

Preferably, the stable structure corresponding to a stroke sequelae-associated substance is one or more selected from:

(1) a stable structure corresponding to a substance causing stroke sequelae in an organism;
(2) a stable structure corresponding to a substance of an organism itself associated with stroke sequelae;
(3) a stable structure corresponding to a substance containing a stroke sequelae-inhibiting structure;
(4) a stable structure corresponding to a substance containing the substance according to any one of (1) to (3) above.

In another preferred embodiment, the stroke sequelae-associated substance is derived from substances in the nervous system.

In another preferred embodiment, the stroke sequelae-associated substance is derived from content in the cranial cavity.

A substance for treatment and/or prevention of stroke sequelae, designed according to the above method.

A method for preparing the above substance for treatment and/or prevention of stroke sequelae, wherein the stable structure corresponding to the substance is prepared by high-temperature carbonization.

The content in the cranial cavity of an organism contain stroke sequelae-associated pathologically changed substances, and are easy to obtain. Therefore, it is preferable to prepare the substance for treatment and/or prevention of stroke sequelae by high-temperature carbonization of the content in the cranial cavity of an organism. If other organs or tissue also contains stroke sequelae-associated pathologically changed substances, it is also preferable to use these stroke sequelae-associated pathologically changed organs or tissue to prepare the substance for treatment and/or prevention of stroke sequelae by high-temperature carbonization. In doing so, complex problems are simplified, and substances for treatment and/or prevention of stroke sequelae can be designed and prepared without knowing the specific pathologically changed structure or the mechanism of pathogenicity. In the present disclosure, it is preferable to use content in the cranial cavity of Caprinae to prepare the substance for treatment and/or prevention of stroke sequelae by high-temperature carbonization.

Use of the above substance for treatment and/or prevention of stroke sequelae in the manufacture of medicaments, health products, food, and food additives.

A method for designing a substance for treatment and/or prevention of cataract, comprising:
using a stable structure corresponding to a cataract-associated substance as the substance for treatment and/or prevention of cataract.

Preferably, the stable structure corresponding to the substance refers to the structure of the substance that is present in a solid state at a high temperature of 500°C or more.

Preferably, the stable structure corresponding to a cataract-associated substance is one or more selected from:

(1) a stable structure corresponding to a substance causing cataract in an organism;
(2) a stable structure corresponding to a substance of an organism itself associated with cataract;
(3) a stable structure corresponding to a substance containing a cataract-inhibiting structure;
(4) a stable structure corresponding to a substance containing the substance according to any one of (1) to (3) above.

In another preferred embodiment, the cataract-associated substance is derived from eyes of an organism.

A substance for treatment and/or prevention of cataract, designed according to the above method.

A method for preparing the above substance for treatment and/or prevention of cataract, wherein the stable structure corresponding to the substance is prepared by high-temperature carbonization.

Eyes of an organism contain cataract-associated pathologically changed substances, and are easy to obtain. Therefore, it is preferable to prepare the substance for treatment and/or prevention of cataract by high-temperature carbonization of eyes of an organism.

Use of the above substance for treatment and/or prevention of cataract in the manufacture of medicaments, health products, food, and food additives.

A method for designing a substance for treatment and/or prevention of xerophthalmia, comprising: using a stable structure corresponding to a xerophthalmia-associated substance as the substance for treatment and/or prevention of xerophthalmia.

Preferably, the stable structure corresponding to the substance refers to the structure of the substance that is present in a solid state at a high temperature of 500°C or more.

Preferably, the stable structure corresponding to a xerophthalmia-associated substance is one or more selected from:

(1) a stable structure corresponding to a substance causing xerophthalmia in an organism;
(2) a stable structure corresponding to a substance of an organism itself associated with xerophthalmia;
(3) a stable structure corresponding to a substance containing a xerophthalmia-inhibiting structure;
(4) a stable structure corresponding to a substance containing the substance according to any one of (1) to (3) above.

In another preferred embodiment, the xerophthalmia-associated substance is one or more selected from: eyes of an organism, immune organs of an organism, and the liver.

A substance for treatment and/or prevention of xerophthalmia, designed according to the above method.

A method for preparing the above substance for treatment and/or prevention of xerophthalmia, wherein the stable structure corresponding to the substance is prepared by high-temperature carbonization.

According to the theory of traditional Chinese medicine, the liver governs the eyes. The xerophthalmia is a liver-associated disease mainly caused by structural imbalance of the liver. Therefore, the liver of an organism contains xerophthalmia-associated pathologically changed substances, and is easy to obtain. Therefore, it is preferable to prepare the substance for treatment and/or prevention of xerophthalmia by high-temperature carbonization of the liver of an organism.

Use of the above substance for treatment and/or prevention of xerophthalmia in the manufacture of medicaments, health products, food, and food additives.

A method for designing a substance for treatment and/or prevention of lacrimation, comprising: using a stable structure corresponding to a lacrimation-associated substance as the substance for treatment and/or prevention of lacrimation.

Preferably, the stable structure corresponding to the substance refers to the structure of the substance that is present in a solid state at a high temperature of 500°C or more.

Preferably, the stable structure corresponding to a lacrimation-associated substance is one or more selected from:

(1) a stable structure corresponding to a substance causing lacrimation in an organism;
(2) a stable structure corresponding to a substance of an organism itself associated with lacrimation;
(3) a stable structure corresponding to a substance containing a lacrimation-inhibiting structure;
(4) a stable structure corresponding to a substance containing the substance according to any one of (1) to (3) above.

In another preferred embodiment, the lacrimation-associated substance is one or more selected from: eyes of an organism, and the liver.

A substance for treatment and/or prevention of lacrimation, designed according to the above method.

According to the theory of traditional Chinese medicine, the liver governs the eyes. Lacrimation is a liver-associated disease mainly caused by structural imbalance of the liver. Therefore, the liver of an organism contains lacrimation-associated pathologically changed substances, and is easy to obtain. Therefore, it is preferable to prepare the substance for treatment and/or prevention of lacrimation by high-temperature carbonization of the liver of an organism.

A method for preparing the above substance for treatment and/or prevention of lacrimation, wherein the stable structure corresponding to the substance is prepared by high-temperature carbonization.

Use of the above substance for treatment and/or prevention of lacrimation in the manufacture of medicaments, health products, food, and food additives.

A method for designing a substance for treatment and/or prevention of hyperthyoidism, comprising:
using a stable structure corresponding to a hyperthyoidism-associated substance as the substance for treatment and/or prevention of hyperthyoidism.

Preferably, the stable structure corresponding to the substance refers to the structure of the substance that is present in a solid state at a high temperature of 500°C or more.

Preferably, the stable structure corresponding to a hyperthyoidism-associated substance is one or more selected from:

(1) a stable structure corresponding to a substance causing hyperthyoidism in an organism;
(2) a stable structure corresponding to a substance of an organism itself associated with hyperthyoidism;
(3) a stable structure corresponding to a substance containing a hyperthyoidism-inhibiting structure;
(4) a stable structure corresponding to a substance containing the substance according to any one of (1) to (3) above.

In another preferred embodiment, the hyperthyoidism-associated substance is derived from substances in the endocrine system.

A substance for treatment and/or prevention of hyperthyoidism, designed according to the above method.

A method for preparing the above substance for treatment and/or prevention of hyperthyoidism, wherein the stable structure corresponding to the substance is prepared by high-temperature carbonization.

Use of the above substance for treatment and/or prevention of hyperthyoidism in the manufacture of medicaments, health products, food, and food additives.

A method for designing a substance for treatment and/or prevention of hypothyoidism, comprising:
using a stable structure corresponding to a hypothyoidism-associated substance as the substance for treatment and/or prevention of hypothyoidism.

Preferably, the stable structure corresponding to the substance refers to the structure of the substance that is present in a solid state at a high temperature of 500°C or more.

Preferably, the stable structure corresponding to a hypothyoidism-associated substance is one or more selected from:

(1) a stable structure corresponding to a substance causing hypothyoidism in an organism;
(2) a stable structure corresponding to a substance of an organism itself associated with hypothyoidism;
(3) a stable structure corresponding to a substance containing a hypothyoidism-inhibiting structure;
(4) a stable structure corresponding to a substance containing the substance according to any one of (1) to (3) above.

In another preferred embodiment, the hypothyoidism-associated substance is derived from substances in the endocrine system.

A substance for treatment and/or prevention of hypothyoidism, designed according to the above method.

A method for preparing the above substance for treatment and/or prevention of hypothyoidism, wherein the stable structure corresponding to the substance is prepared by high-temperature carbonization.

Use of the above substance for treatment and/or prevention of hypothyoidism in the manufacture of medicaments, health products, food, and food additives.

A method for designing a substance for treatment and/or prevention of constipation, comprising:
using a stable structure corresponding to a constipation-associated substance as the substance for treatment and/or prevention of constipation.

Preferably, the stable structure corresponding to the substance refers to the structure of the substance that is present in a solid state at a high temperature of 500°C or more.

Preferably, the stable structure corresponding to a constipation-associated substance is one or more selected from:

(1) a stable structure corresponding to a substance causing constipation in an organism;
(2) a stable structure corresponding to a substance of an organism itself associated with constipation;
(3) a stable structure corresponding to a substance containing a constipation-inhibiting structure;
(4) a stable structure corresponding to a substance containing the substance according to any one of (1) to (3) above.

In another preferred embodiment, the constipation-associated substance is one or more selected from: substances in the nervous system, the spleen of an organism, and the intestinal tract of an organism.

Constipation is present in the large intestine, and may be due to various causes such as an irregular diet, mood disorders, and negative external substance. In traditional Chinese medicine, spleen weakness and exhaustion of qi and blood, or body fluid deficiency and blood dryness, or excess heat and blood stasis, or qi stagnation and blood stasis, lead to poor circulation of qi and blood in the *Yangming* meridian and impaired large intestinal qi, resulting in constipation. The constipation-associated pathologically changed substances are mainly involved in the intestine and spleen of an organism.

A substance for treatment and/or prevention of constipation, designed according to the above method.

A method for preparing the above substance for treatment and/or prevention of constipation, wherein the stable structure corresponding to the substance is prepared by high-temperature carbonization.

The substances in the intestinal tract and spleen of an organism contain constipation-associated pathologically changed substances, and are easy to obtain. Therefore, it is preferable to prepare the substance for treatment and/or prevention of constipation by high-temperature carbonization of the substances in the intestinal tract and spleen of an organism. If other organs or tissue also contains constipation-associated pathologically changed substances, it is also preferable to use these constipation-associated pathologically changed organs or tissue to prepare the substance for treatment and/or prevention of constipation by high-temperature carbonization. In doing so, complex problems are simplified, and substances for treatment and/or prevention of constipation can be designed and prepared without knowing the specific pathologically changed structure or the mechanism of pathogenicity. In the present disclosure, it is preferable to use intestinal tract and/or spleen of swine to prepare the substance for treatment and/or prevention of constipation by high-temperature carbonization.

Use of the above substance for treatment and/or prevention of constipation in the manufacture of medicaments, health products, food, and food additives.

A method for designing a substance for treatment and/or prevention of sexual dysfunction, comprising: using a stable structure corresponding to a sexual dysfunction-associated substance as the substance for treatment and/or prevention of sexual dysfunction.

Preferably, the stable structure corresponding to the substance refers to the structure of the substance that is present in a solid state at a high temperature of 500°C or more.

Preferably, the stable structure corresponding to a sexual dysfunction-associated substance is one or more selected from:

(1) a stable structure corresponding to a substance causing sexual dysfunction in an organism;
(2) a stable structure corresponding to a substance of an organism itself associated with sexual dysfunction;
(3) a stable structure corresponding to a substance containing a sexual dysfunction-inhibiting structure;
(4) a stable structure corresponding to a substance containing the substance according to any one of (1) to (3) above.

In another preferred embodiment, the sexual dysfunction-associated substance is one or more selected from: substances in the nervous system, substances in the endocrine system, and the reproductive organ of an organism.

A substance for treatment and/or prevention of sexual dysfunction, designed according to the above method.

A method for preparing the above substance for treatment and/or prevention of sexual dysfunction, wherein the stable structure corresponding to the substance is prepared by high-temperature carbonization.

Use of the above substance for treatment and/or prevention of sexual dysfunction in the manufacture of medicaments, health products, food, and food additives.

A method for designing a substance for treatment and/or prevention of degenerative bone diseases, comprising: using a stable structure corresponding to a degenerative bone disease associated substance as the substance for treatment and/or prevention of degenerative bone diseases.

Preferably, the stable structure corresponding to the substance refers to the structure of the substance that is present in a solid state at a high temperature of 500°C or more.

Preferably, the stable structure corresponding to a degenerative bone disease associated substance is one or more selected from:

(1) a stable structure corresponding to a substance causing degenerative bone diseases in an organism;
(2) a stable structure corresponding to a substance of an organism itself associated with degenerative bone diseases;
(3) a stable structure corresponding to a substance containing a degenerative bone diseases-inhibiting structure;
(4) a stable structure corresponding to a substance containing the substance according to any one of (1) to (3) above.

In another preferred embodiment, the degenerative bone diseases associated substance is derived from the locomotor system, substances in the endocrine system, preferably the joints of an organism.

A substance for treatment and/or prevention of degenerative bone diseases, designed according to the above method.

A method for preparing the above substance for treatment and/or prevention of degenerative bone diseases, wherein the stable structure corresponding to the substance is prepared by high-temperature carbonization.

Use of the above substance for treatment and/or prevention of degenerative bone diseases in the manufacture of medicaments, health products, food, and food additives.

A method for designing a substance for treatment and/or prevention of urticaria, comprising:

using a stable structure corresponding to an urticaria-associated substance as the substance for treatment and/or prevention of urticaria.

Preferably, the stable structure corresponding to the substance refers to the structure of the substance that is present in a solid state at a high temperature of 500°C or more.

Urticaria is a skin disease characterized by wheal and angioedema, with various and complex causes. The foreign substances that cause urticaria such as castor beans, drugs and other substances are substance associated with diseases. Urticaria is a symptom, that is, an external manifestation of structural imbalance. Different substances can cause the same or similar structural imbalance, and the possible structures in these substances causing urticaria in an organism are the same or similar. Therefore, using only the stable structure corresponding to a known substance therein to act on the biological structure system can accomplish the supporting of "*the positive*" with "*the external negative*" according to the present application, thanks to the self-adapting, self-organizing, and structural stability-maintaining function of the biological structure system. According to the method for designing a substance for treatment and/or prevention of urticaria of the present disclosure, the stable structure corresponding to castor bean is preferably used in the present disclosure as the substance for treatment and/or prevention of urticarial, because substances like ricin, ricinine, castor allergen and hemagglutinin in castor bean can cause urticaria, and the symptoms of disease are typical.

Preferably, the stable structure corresponding to an urticaria-associated substance is one or more selected from:

(1) a stable structure corresponding to a substance causing urticaria in an organism;
(2) a stable structure corresponding to a substance of an organism itself associated with urticaria;
(3) a stable structure corresponding to a substance containing an urticaria-inhibiting structure;
(4) a stable structure corresponding to a substance containing the substance according to any one of (1) to (3) above.

A substance for treatment and/or prevention of urticaria, designed according to the above method.

A method for preparing the above substance for treatment and/or prevention of urticaria, wherein the stable structure corresponding to the substance is prepared by high-temperature carbonization.

Preferably, the substance for treatment and/or prevention of urticarial is prepared by high-temperature carbonization of caster beans.

Use of the above substance for treatment and/or prevention of urticaria in the manufacture of medicaments, health products, food, and food additives.

A method for designing a substance for treatment and/or prevention of conjunctivitis, comprising:

using a stable structure corresponding to a conjunctivitis-associated substance as the substance for treatment and/or prevention of conjunctivitis.

Preferably, the stable structure corresponding to the substance refers to the structure of the substance that is present in a solid state at a high temperature of 500°C or more.

Conjunctivitis is redness or inflammation on the transparent membrane covering the eyeball and a part the membrane inside the inner eyelid. Allergens, chemical agents, and underlying diseases can cause redness of eyes. Therefore, from the perspective of Western medicine, the allergens that cause conjunctivitis are substances associated with conjunctivitis. From the structure point of view, the root cause is the imbalance between the biological structure system and the structure of the allergens upon interactions. From the perspective of traditional Chinese medicine, the *Neijing* says that "the liver is physiologically connected to the eyes" via the foot *Jueyin* channel, so the eyes are most closely associated with the liver meridian. Alhtough "all meridians relate to the eyes" and "the essence of the five ZANG-organs and six FU-organs are all upwards converged on the eyes" indicating that the eyes have extensive connections with the meridians and viscera, it is most closely related to the foot *Jueyin* channel, and thus reference to "eyes" is always intimated with the foot *Jueyin*. If the liver blood is sufficient, the eyes will be bright, and the vision will be clear; if the liver blood is insufficient, the eyes will be malnutritional, dry and dim, and the vision will be unclear or night blind; if the liver meridian is invaded by wind heat, the eyes will be red and itchy; if the liver is inflamed, the eyes are red and produce cataract; if the liver *Yang* is hyperactive, dizziness is felt in the head and eyes; if the liver wind is stirred, the eyes have squint and an upward vision. Therefore, from the perspective of traditional Chinese medicine, the cause of conjunctivitis lies in the liver. From a structural perspective, the root cause is imbalance of certain structures in the structure system of the liver of an organism, which leads to structural imbalance between the biological structure system and the allergen structure, resulting in conjunctivitis, especially allergic conjunctivitis. The balance of the biological structure system is dynamic and is related to the external structural environment. For example, for humans, some substances easily generally cause allergic conjunctivitis in humans, such as *Artemisia desertorum,* but not in organisms such as swine, bovine and Caprinae which are not allergic to *Artemisia desertorum,* which means that the structural imbalance of the liver is relative, and the liver structure of swine, bovine and Caprinae contains a structure that interacts with the structure of *Artemisia desertorum* to prevent allergic conjunctivitis in these organisms, i.e. a structure that inhibits the cause of conjunctivitis.

Preferably, the stable structure corresponding to a substance associated with conjunctivitis is one or more selected from:

(1) a stable structure corresponding to a substance causing conjunctivitis in an organism;

The imbalance of the biological structure system includes the imbalance between the biological structure system itself and a non-self-structure. In the present disclosure, the biological structure system itself is called "*the positive*", and the external structure that causes imbalance of the biological structure system is called "*the external negative*". Substances causing conjunctivitis in an organism are "*the external negative*", and will result in structural imbalance causing symptoms of allergic rhinitis in the organism when acting on the biological structure system. The essence is that the biological structure system having allergic conjunctivitis fails to reach a dynamic balance with "*the external negative*". According to the present disclosure, the stable structure corresponding to "*the external negative*" is used as an external structure to act on the biological structure system. Due to the self-adapting, self-organizing and self-stabilizing function of the biological structure system, the biological structure system will regulate the expression of genes of structures that interact with an external structure according to the structural information from the external structure, so as to achieve a balance with the external structure, thereby establishing a balance between the biological structure system and "*the external negative*", i.e. supporting the "*positive*" by the "*external negative*".

(2) a stable structure corresponding to a conjunctivitis-associated substance in an organism itself;

The imbalance of the biological structure system includes the imbalance of the internal structure in the biological structure system, that is, the pathological change of the internal structure, which is often the imbalance of structural interaction. For example, for a certain structure in the biological structure system, under-expression of genes of structures restricting it or over-expression of genes of structures promoting it will break the balance between structures. The present disclosure refers to the imbalanced structure in the biological structure system as "*the internal negative*". Conjunctivitis is associated with an imbalanced structure in the liver, that is, the imbalanced structure in the liver that causes conjunctivitis is "*the internal negative*", a conjunctivitis -associated substance of the organism itself. The stable structure corresponding to "*the internal negative*" is used as an external structure to act on the biological structure system. Due to the self-adapting, self-organizing and self-stabilizing function of the biological structure system, the biological structure system will regulate the expression of genes of structures that interact with the external structure according to the structural information from the external structure, so as to achieve a balance with the external structure, thus realizing restoration of a balance of the imbalanced internal structure in the biological structure system and treatment of diseases, i.e. supporting the "*positive*" by the "*internal negative*".

Imbalanced internal structures in the biological structure system have corresponding balanced structures; for each structure, there is a structure interacting with it; and these balanced structures are also referred to as "*the positive*" in the present disclosure. Conjunctivitis is associated with structural imbalance in the liver, and the imbalanced structure in the liver is "*the internal negative*". The liver of healthy organisms without conjunctivitis is "*the positive*", and the stable structure corresponding to it is used as an external structure to act on the biological structure system. Due to the self-adapting, self-organizing and self-stabilizing function of the biological structure system, the biological structure system will regulate the expression of genes of structures that interact with the external structure to a normal level according to the structural information from each structure in the external structure, so that the imbalanced structure restores the balance, i.e. supporting the "*positive*" by the "*positive*".

(3) a stable structure corresponding to a substance containing a conjunctivitis-inhibiting structure;

A substance capable of causing a disease may not cause the disease in a certain organism, in which case the structure system of the certain organism can achieve a balance with the structure of the disease-causing substance upon interaction, that is to say, the disease-associated structure in the certain organism has a structure that interacts with the structure of the disease-causing substance and prevents the disease in the organism, and such a structure is not isolated but in interactions with other structures in the biological structure system, thereby reaching a balance. Some substance generally causes allergic conjunctivitis in human, but not in certain organisms such as swine, bovine and Caprinae. Therefore, the stable structure corresponding to the liver of the organism in which conjunctivitis would not be caused is used as an external structure to act on the biological structure system in need of treatment of this disease. Due to the self-adapting, self-organizing and self-stabilizing function of the biological structure system, the biological structure system will regulate the genes or expression of genes of structures that interact with the external structure according to the structural information from each structure in the external structure, so that a balance is achieved between the biological structure system and the structure of the disease-causing substance, which is also supporting the "*positive*" by the "*positive*".

(4) a stable structure corresponding to a substance containing the substance according to any one of (1) to (3) above.

A substance for treatment and/or prevention of conjunctivitis, designed according to the above method.

A method for preparing the above substance for treatment and/or prevention of conjunctivitis, wherein the stable structure corresponding to the substance is prepared by high-temperature carbonization.

The substance for treatment and/or prevention of conjunctivitis is prepared by high-temperature carbonization of a substance containing a conjunctivitis-associated substance, because such a substance is easily available and makes the problem simpler. For allergens that externally cause allergic conjunctivitis, allergen-containing substances are used directly without the need to isolate the specific allergens or the need to know the specific mechanism of allergies in an organism. For instance, for allergic conjunctivitis caused by pollen, there is no need to know or isolate the specific allergy-causing substances in the pollen, and the pollen is directly subjected to high-temperature carbonization to make a desensitizing substance for treatment and/or prevention of allergic conjunctivitis, so as to support the "*positive*" with the "*external negative*". Following the same principle, the liver, as a conjunctivitis -associated substance of an organism, preferably of swine, bovine, Caprinae or primate, may be directly prepared into a substance for treatment and/or prevention of conjunctivitis by high-temperature carbonization, so as to support the "*positive*" with the "*external negative*".

Use of the above substance for treatment and/or prevention of conjunctivitis in the manufacture of medicaments, health products, food, and food additives.

A method for designing a substance for treatment and/or prevention of asthma, comprising:

using a stable structure corresponding to an asthma-associated substance as the substance for treatment and/or prevention of asthma.

Preferably, the stable structure corresponding to the substance refers to the structure of the substance that is present in a solid state at a high temperature of 500°C or more.

From the perspective of Western medicine, asthma is a chronic airway inflammation involving a variety of cells and cell components, and inhalation of allergens is the major provoking factor for asthma. Therefore, the allergens that cause asthma are substances associated therewith. From the structural point of view, the root cause is imbalance between the biological structure system and the structure of allergens upon interaction. From the perspective of traditional Chinese medicine, the main symptoms of asthma are wheezing and gasping, and the main pathogenesis of asthma is abnormal inhalation and exhalation of the lung caused by retained phlegm in the lung. The disease is located in the lung and mainly involves the lung meridian and the stomach meridian. Asthma is caused by the internal negatives and induced by external factors such that the lung qi is reversed upwards and the dispersing and descending function of the lung is impaired. People with pyretic pulmonary diseases feel unconscious first, then show vellus hair, aversion to wind and cold, yellow tongue, body fever, and heat conflicting which causes dyspnea with cough. From the perspective of traditional Chinese medicine, the cause of asthma lies in the lung. From a structural perspective, the root cause is imbalance of certain structures in the structure system of the lung of an organism, which leads to structural imbalance between the biological structure system and the allergen structure, resulting in asthma, especially allergic asthma. The balance of the biological structure system is dynamic and is related to the external structural environment. For example, for humans, some substances easily generally cause allergic asthma in humans, such as *Artemisia desertorum*, but not in organisms such as swine, bovine and Caprinae which are not allergic to *Artemisia desertorum,* which means that the structural imbalance of the liver is relative, and the liver structure of swine, bovine and Caprinae contains a structure that interacts with the structure of *Artemisia desertorum* to prevent allergic asthma in these organisms, i.e. a structure that inhibits the cause of asthma.

Preferably, the stable structure corresponding to a substance associated with asthma is one or more selected from:

(1) a stable structure corresponding to a substance causing asthma in an organism;

The imbalance of the biological structure system includes the imbalance between the biological structure system itself and a non-self structure. In the present disclosure, the biological structure system itself is called "*the positive*", and the external structure that causes imbalance of the biological structure system is called "*the external negative*". Substances causing asthma in an organism are "*the external negative*", and will result in structural imbalance causing symptoms of allergic rhinitis in the organism when acting on the biological structure system. The essence is that the biological structure system having allergic asthma fails to reach a dynamic balance with "*the external negative*". According to the present disclosure, the stable structure corresponding to "*the external negative*" is used as an external structure to act on the biological structure system. Due to the self-adapting, self-organizing and self-stabilizing function of the biological structure system, the biological structure system will regulate the expression of genes of structures that interact with an external structure according to the structural information from the external structure, so as to achieve a balance with the external structure, thereby establishing a balance between the biological structure system and "*the external negative*", i.e. supporting the "*positive*" by the "*external negative*"*.

(2) a stable structure corresponding to an asthma-associated substance of an organism itself;

The imbalance of the biological structure system includes the imbalance of the internal structure in the biological structure system, that is, the pathological change of the internal structure, which is often the imbalance of structural interaction. For example, for a certain structure in the biological structure system, under-expression of genes of structures restricting it or over-expression of genes of structures promoting it will break the balance between structures. The present disclosure refers to the imbalanced structure in the biological structure system as "*the internal negative*". Asthma is associated with an imbalanced structure in the lung, that is, the imbalanced structure in the lung that causes asthma is "*the internal negative*", an asthma -associated substance of the organism itself. The stable structure corresponding to "*the internal negative*" is used as an external structure to act on the biological structure system. Due to the self-adapting, self-organizing and self-stabilizing function of the biological structure system, the biological structure system will regulate the expression of genes of structures that interact with the external structure according to the structural information from the external structure, so as to achieve a balance with the external structure, thus realizing restoration of a balance of the imbalanced internal structure in the biological structure system and treatment of diseases, i.e. supporting the "*positive*" by the "*internal negative*".

Imbalanced internal structures in the biological structure system have corresponding balanced structures; for each structure, there is a structure interacting with it; and these balanced structures are also referred to as "*the positive*" in the present disclosure. Asthma is associated with structural imbalance in the lung, and the imbalanced structure in the lung is "*the internal negative*". The lung of healthy organisms without asthma is "*the positive*", and the stable structure corresponding to it is used as an external structure to act on the biological structure system. Due to the self-adapting, self-organizing and self-stabilizing function of the biological structure system, the biological structure system will regulate the expression of genes of structures that interact with the external structure to a normal level according to the structural information from each structure in the external structure, so that the imbalanced structure restores the balance, i.e. supporting the "*positive*" by the "*positive*".

(3) a stable structure corresponding to a substance containing an asthma-inhibiting structure;

A substance capable of causing a disease may not cause the disease in a certain organism, in which case the structure system of the certain organism can achieve a balance with the structure of the disease-causing substance upon interaction, that is to say, the disease-associated structure in the certain organism has a structure that interacts with the structure of the disease-causing substance and prevents the disease in the organism, and such a structure is not isolated but in interactions with other structures in the biological structure system, thereby reaching a balance.

Some substance generally causes allergic asthma in human, but not in certain organisms such as swine, bovine and Caprinae. Therefore, the stable structure corresponding to the lung of the organism in which asthma would not be caused is used as an external structure to act on the biological structure system in need of treatment of this disease. Due to the self-adapting, self-organizing and self-stabilizing function of the biological structure system, the biological structure system will regulate the genes or expression of genes of structures that interact with the external structure according to the structural information from each structure in the external structure, so that a balance is achieved between the biological structure system and the structure of the disease-causing substance, which is also supporting the "*positive*" by the "*positive*".

(4) a stable structure corresponding to a substance containing the substance according to any one of (1) to (3) above.

A substance for treatment and/or prevention of asthma, designed according to the above method.

A method for preparing the above substance for treatment and/or prevention of asthma, wherein the stable structure corresponding to the substance is prepared by high-temperature carbonization.

The substance for treatment and/or prevention of asthma is prepared by high-temperature carbonization of a substance containing an asthma-associated substance, because such a substance is easily available and makes the problem simpler. For allergens that externally cause allergic asthma, allergen-containing substances are used directly without the need to isolate the specific allergens or the need to know the specific mechanism of allergies in an organism. For instance, for allergic asthma caused by pollen, there is no need to know or isolate the specific allergy-causing substances in the pollen, and the pollen is directly subjected to high-temperature carbonization to make a desensitizing substance for treatment and/or prevention of allergic asthma, so as to support the "*positive*" with the "*external negative*". Following the same principle, the lung, as an asthma-associated substance of an organism, preferably of swine, bovine, Caprinae or primate, may be directly prepared into a substance for treatment and/or prevention of asthma by high-temperature carbonization, so as to support the "*positive*" with the "*positive*".

Use of the above substance for treatment and/or prevention of asthma in the manufacture of medicaments, health products, food, and food additives.

A method for designing a substance for treatment and/or prevention of ankylosing spondylitis, comprising: using a stable structure corresponding to an ankylosing spondylitis-associated substance as the substance for treatment and/or prevention of ankylosing spondylitis.

Preferably, the stable structure corresponding to the substance refers to the structure of the substance that is present in a solid state at a high temperature of 500°C or more.

Preferably, the stable structure corresponding to an ankylosing spondylitis-associated substance is one or more selected from:

(1) a stable structure corresponding to a substance causing ankylosing spondylitis in an organism;

(2) a stable structure corresponding to a substance of an organism itself associated with ankylosing spondylitis;

(3) a stable structure corresponding to a substance containing an ankylosing spondylitis-inhibiting structure;

(4) a stable structure corresponding to a substance containing the substance according to any one of (1) to (3) above.

Preferably, the substances containing an ankylosing spondylitis-associated substance include organs or tissue where the ankylosing spondylitis-associated substance is present, preferably the connective tissue of an organism according to the present disclosure.

A substance for treatment and/or prevention of ankylosing spondylitis, designed according to the above method.

A method for preparing the above substance for treatment and/or prevention of ankylosing spondylitis, wherein the stable structure corresponding to the substance is prepared by high-temperature carbonization.

Use of the above substance for treatment and/or prevention of ankylosing spondylitis in the manufacture of medicaments, health products, food, and food additives.

A method for designing a substance for treatment and/or prevention of infectious diseases, comprising: using a stable structure corresponding to an infectious disease associated substance as the substance for treatment and/or prevention of infectious disease.

Preferably, the stable structure corresponding to the substance refers to the structure of the substance that is present in a solid state at a high temperature of 500°C or more.

Preferably, the stable structure corresponding to an infectious disease associated substance is one or more selected from:

(1) a stable structure corresponding to a substance causing infectious disease in an organism;

(2) a stable structure corresponding to a substance of an organism itself associated with infectious disease;

(3) a stable structure corresponding to a substance containing an infectious disease-inhibiting structure;

(4) a stable structure corresponding to a substance containing the substance according to any one of (1) to (3) above.

In another preferred embodiment, the infectious diseases associated substance is one or more selected from bacteria, fungi, viruses, viroids, and parasites.

A substance for treatment and/or prevention of infectious diseases, designed according to the above method.

A method for preparing the above substance for treatment and/or prevention of infectious diseases, wherein the stable structure corresponding to the substance is prepared by high-temperature carbonization.

Use of the above substance for treatment and/or prevention of infectious diseases in the manufacture of medicaments, health products, food, and food additives.

In the present disclosure, as regards the stable structure corresponding to the substance prepared by high-temperature carbonization, if the substance is derived from an organism, then the organism is preferably swine, bovine, Caprinae, or primate.

The essence of health is the dynamic balance of the structure system. This dynamic balance is the result of the interactions between structures in the structure system. The most basic interactions are promotion and restriction. In the biological structure system, if a structure does not have another structure promoting it, this structure will be deficient, and if it does not have another structure restricting it, this structure will be excessive, both resulting in imbalance in the structure system. The essence of a disease is imbalance of the biological structure system. It can be seen from the above technical solutions that the method for treating or preventing diseases according to the present disclosure uses the stable structure of a substance associated with disease as an external structure to act on the biological structure system (such as by oral administration), and the biological structure system regulates genes or expression of genes by recognition and transmission of the structural information from the external structure and by its self-adapting and self-organizing function, so as to treat or prevent diseases associated with the substance.

It can be seen from the above technical solutions that the substance for treatment and/or prevention of diseases, the method for designing the substance, and the method for preparing the substance according to the present disclosure have the following beneficial effects.

1. The methods for designing and preparing the substance for treatment and/or prevention of diseases according to the present disclosure do not focus on the specific structure of the disease-causing substance or the specific mechanism of pathogenicity, but on using a stable structure corresponding to a substance identical or similar to the disease-causing external or internal substance as an external structure to act on the biological structure system. The external structure can make the organism to eliminate, alleviate, or prevent the diseases by self-adaption and self-organization of structures, thereby being universally applicable.

2. The present disclosure combines the specific structure studied by Western medicine and the macroscopic theory established by traditional Chinese medicine to treat and/or prevent diseases from the perspective of the system of an organism. Relatively speaking, the system is limited, while the disease is infinite, because different pathological changes of the same organ will produce different diseases, different individuals with the same disease show symptoms not completely the same. Humans are the same as each other from a systemic point of view, according to both Western medicine and traditional Chinese medicine, and humans are even substantially similar to swine, bovine, Caprinae, and primate. Therefore, the substance for treatment and/or prevention of diseases is designed according to the system. For diseases caused by internal pathogenesis of an organism, one only needs to know the specific pathologically changed cells, tissue or organs, without the need to investigate the mechanism of pathogenicity or the specific symptoms of the diseases. What is needed is to use the stable structure corresponding to the system where the substance associated with disease is present, or cells, tissue or organs in the system, as an external structure to act on the biological structure system, and then the biological structure system can establish a balance with the structure of the external substance or restore the internal balance in the biological structure system according to self-adaptation and self-organization of structures, thereby eliminate, alleviate, or prevent diseases.

3. The substance for treatment and/or prevention of diseases, the method for designing the substance, and the method for preparing the substance according to the present disclosure circumvent the need to study the disease mechanism, greatly save time, manpower, material resources, and financial resources, significantly shorten the drug R&D cycle, and lower the cost of treatment of diseases, representing an extreme simplification of drug research and development, and diagnosis and treatment of diseases.

**Detailed description of invention**

The present disclosure relates to a substance for treatment and/or prevention of diseases, the method for designing the substance, and the method for preparing the substance. Those skilled in the art can learn from the content of the specification to use different materials or improve the process or use other similar process for preparation, which are all considered to be included in the scope of the present disclosure. It is particularly noteworthy that all similar replacements and modifications are obvious to those skilled in the art, and they are all deemed to be included in the scope of the present disclosure. The method and product of the present disclosure have been described with reference to preferred examples, and it is obvious that those skilled in the art can make modifications or appropriate changes and combinations to the methods described herein without departing from the content, spirit and scope of the present disclosure to carry out and apply the technique of the present disclosure.

In order to provide further understanding of the present disclosure, the technical solutions in examples of the present disclosure will be clearly and completely described below in conjunction with the examples of the present disclosure. Apparently, the examples described below are only a part of examples of the present invention rather than all of them. Based on the examples of the present disclosure, all other examples obtained by those of ordinary skill in the art without paying creative work shall fall within the protection scope of the present invention.

The embodiments are as follows.

**Example** 1 Substance for treatment of depression and method for preparing the substance

Depression is a chronic recurrent disease having both affective and physical symptoms. The neurotransmitter serotonin in patients' brain is disordered or deficient, resulting in significant and lasting depressed mood. Therefore, depression mainly involves the nervous system and endocrine system. The content in the cranial cavity of an organism contain depression-associated substances, and are easy to obtain. Therefore, in this example, the substance for treatment or prevention of depression was prepared by high-temperature carbonization of the content in the cranial cavity of Caprinae. If other organs or tissue, such as the adrenal glands, also contains depression-associated pathologically changed substances, it is also preferable to use these depression-associated pathologically changed organs or tissue to prepare the substance for treatment and/or prevention of depression by high-temperature carbonization. In doing so, complex problems are simplified, and substances for treatment and/or prevention of depression can be designed and prepared without knowing the specific pathologically changed structure or the mechanism of pathogenicity.

In this example, content in the cranial cavity of Caprinae were selected to prepare the substance for treatment and/or prevention of depression by high-temperature carbonization.

**Example** 2 Clinical study with the substance prepared in Example 1 against depression

This example used the substance prepared in Example 1 for efficacy verification, as specifically shown below.

1. Clinical data: 60 cases (in total) of outpatients and inpatients with depression were enrolled, and randomly divided into a treatment group of 30 patients and a control group of 30 patients. There was no significant difference between the two groups in clinical data. There was no statistically significant difference between the two groups in age, gender, course of disease, and complication (P>0.05), and they were comparable.

2. Diagnosis criteria: The diagnosis criteria for depression from *The Diagnostic and Statistical Manual of Mental Disorders*.

3. Treatment method: Patients in the treatment group orally took the substance for treatment of depression prepared in Example 1, 3 g per time, once a day, for 7 consecutive days. Patients in the control group were treated with fluoxetine, the initial and treatment doses both being 20 mg/day, for a course of 4 weeks.

4. Efficacy evaluation criteria: After 4 weeks of treatment, the patients were scored by a psychiatrist based on the Hamilton Depression Rating Scale HAMD-17. "Cured" means a HAMD-17 total score <7; "effective" means a HAMD-17 total score of 8-17; and "ineffective" means a HAMD-17 total score>17.

$$\text{Total effective rate} = \text{cured rate} + \text{effective rate}.$$

5. Efficacy results: After treatment, 27 patients in the treatment group were "cured" or showed "effective", with a total effective rate of 90.00%, which is significantly (P <0.05) higher than that (76.67%) of the control group.

Table 1 Comparison of the effectiveness in patients with depression after treatment (as Examples)

| Group | Number of patients | Cured | Effective | Ineffective | Total effective rate (%) |
|---|---|---|---|---|---|
| Treatment | 30 | 20 | 7 | 3 | 90.00% |
| Control | 30 | 9 | 14 | 7 | 76.67% |

**Example** 3 Substance for treatment of menoxenia and method for preparing the substance

Menstruation is formed after the hormones secreted by the ovary act on the endometrium. The secretion of hormones by the ovary is controlled by the hormones released by the pituitary gland and hypothalamus. Therefore, abnormalities in the function of any of the ovary, pituitary gland, and hypothalamus will affect menstruation. Therefore, menoxenia mainly involves the endocrine system. The content in the cranial cavity of an organism contain the pituitary gland and hypothalamus, and are easy to obtain. Therefore, in this example, the substance for treatment and/or prevention of menoxenia was prepared by high-temperature carbonization of the content in the cranial cavity of Caprinae. If other organs or tissue, such as the ovary or spleen, also contains menoxenia-associated pathologically changed substances, it is also preferable to use these menoxenia-associated pathologically changed organs or tissue to prepare the substance for treatment and/or prevention of menoxenia by high-temperature carbonization. In doing so, complex problems are simplified, and substances for treatment and/or prevention of menoxenia can be designed and prepared without knowing the specific pathologically changed structure or the mechanism of pathogenicity.
In this example, content in the cranial cavity of Caprinae were selected to prepare the substance for treatment or prevention of menoxenia by high-temperature carbonization.

**Example** 4: Clinical study with the substance prepared in Example 3 against menoxenia

1. Clinical data: 60 cases (in total) of outpatients and inpatients with menoxenia were enrolled, and randomly divided into a treatment group of 30 patients and a control group of 30 patients. There was no significant difference between the two groups in clinical data. There was no statistically significant difference between the two groups in age, course of disease, and complication (P>0.05), and they were comparable.

2. Diagnosis criteria: Symptoms include disorders in the menstrual cycle and menstrual period, excessive or insufficient bleeding during menstruation, and abdominal pain before and after menstruation.

3. Treatment method: Patients in the treatment group orally took the substance for treatment of menoxenia prepared in Example 3, at a dose of 3 g, once a day, started on day 5 since menstruation, for 7 consecutive days. Patients in the control group were orally given clomifene citrate capsules (NMPAApproval No. H31021107), 50 mg/day, started on day 5 since menstruation, for 2 consecutive months.

4. Efficacy evaluation criteria: The efficacy evaluation is based on the relevant standards in *Guiding Principles for Clinical Research of New Chinese Medicines.* "Cured" means that clinical symptoms have disappeared, the menstrual cycle returns to $28\pm4$ days after treatment, and does not recur after stopping the drug; "markedly effective" means that clinical symptoms have been significantly alleviated, and the menstrual cycle returns to $28\pm8$ days after treatment, and does not recur after stopping the drug; "effective" means that clinical symptoms have been alleviated, the menstrual cycle has been improved after treatment, and recurs after stopping the drug; "ineffective" means that clinical symptoms have not been improved, and the menstrual cycle has not been improved after treatment, and recurs after stopping the drug.

$$\text{Total effective rate} = \text{cured rate} + \text{effective rate}.$$

5. Treatment results: The total effective rates of the treatment group and the control group were 86.67% and 66.67%, respectively, with a significant difference (P<0.05), indicating that the treatment group showed a better result than the control group.

Table 2 Comparison of the effective rates between the two groups after treatment (as Examples)

| Group | Number of patients | Cured | Markedly effective | Effective | Ineffective | Total effective rate (%) |
|---|---|---|---|---|---|---|
| Treatment | 30 | 8 | 10 | 8 | 4 | 86.67% |
| Control | 30 | 0 | 10 | 10 | 0 | 66.67% |

**Example** 5 Substance for treatment of stroke sequelae and method for preparing the substance

"Stroke" is also called "apoplexy" or "cerebrovascular accident". It is an acute cerebrovascular disease, which is a group of diseases with brain tissue damage due to sudden rupture of blood vessels in the brain or block of blood from flowing into the brain. The stroke sequelae refer to the conditions left after the onset of the acute cerebrovascular disease, mainly manifested in hemiplegia, numbness, crooked mouth and eyes, and aphasia. Therefore, the stroke sequelae mainly involve the nerve system. The content in the cranial cavity of an organism contain stroke sequelae-associated substances, and are easy to obtain. Therefore, in this example, the substance for treatment or prevention of stroke sequelae was prepared by high-temperature carbonization of the content in the cranial cavity of Caprinae. If other organs or tissue also contains stroke sequelae-associated pathologically changed substances, it is also preferable to use these stroke sequelae-associated pathologically changed organs or tissue to prepare the substance for treatment and/or prevention of stroke sequelae by high-temperature carbonization. In doing so, complex problems are simplified, and substances for treatment and/or prevention of stroke sequelae can be designed and prepared without knowing the specific pathologically changed structure or the mechanism of pathogenicity.
In this example, content in the cranial cavity of Caprinae were used to prepare the substance for treatment or prevention of stroke sequelae by high-temperature carbonization.

**Example** 6: Clinical study with the substance prepared in Example 5 against stroke sequelae

This example used the substance prepared in Example 5 for clinical studies, as specifically shown below.

1. Clinical data: 32 cases (in total) of outpatients and inpatients with stroke sequelae were enrolled, and randomly divided into a treatment group of 16 patients and a control group of 16 patients. There was no significant difference between the two groups in clinical data. There was no statistically significant difference between the two groups in age, gender, course of disease, and complication (P>0.05), and they were comparable.

2. Diagnosis criteria: The diagnosis criteria for stroke from the Western medicine reference *Key points in diagnosis of various cerebrovascular diseases.*

3. Treatment method: Patients in the treatment group orally took the substance for treatment of stroke sequelae prepared in Example 5, at a dose of 3 g, once a day, for 7 consecutive days. Patients in the control group were treated with a Western medicine regime: the aspirin enteric-coated tablets from Shijiazhuang KANGLI pharmaceuticals, orally taken, 100 mg per time, once per day, methycobal from Eisai China, orally taken, 0.5 mg per time, 3 times per day, and the isosorbide mononitrate tablets from Lunan Better pharmaceutical Co., Ltd., orally taken, 20 mg per time, once per day, for 2 consecutive months.

4. Efficacy evaluation criteria: "Markedly effective" means complete or substantial improvement in symptoms after treatment, and recovery of limb function, language function, and living ability; "effective" means relief of symptoms after treatment, and improvement in limb function, language function, and living ability; "ineffective" means failure to achieve the above standards after treatment.

$$\text{Total effective rate} = \text{markedly effective rate} + \text{effective rate}.$$

5. Efficacy results: As shown in Table 3, after treatment, 15 patients in the treatment group were "cured" or showed "effective", with a total effective rate of 93.75%, which is significantly ($P < 0.05$) higher than the total effective rate 68.75% of the control group.

Table 3 Comparison of the effectiveness in patients with stroke sequelae after treatment

| Group | Number of patients | Markedly effective | Effective | Ineffective | Total effective rate (%) |
|---|---|---|---|---|---|
| Treatment | 16 | 9 | 6 | 1 | 93.75% |
| Control | 16 | 4 | 7 | 5 | 68.75% |

Example 7: Substance for treatment of degenerative osteoarthropathy and method for preparing the substance

Degenerative osteoarthropathy, also known as osteoarthritis, degenerative arthritis, senile arthritis, or hypertrophic arthritis, is a degenerative disease with degenerative damage to articular cartilage and reactive hyperplasia at joint edges and subchondral bone caused by various factors such as aging, obesity, strain, trauma, joint congenital abnormalities, and joints deformity. Therefore, degenerative osteoarthropathy mainly involves the locomotor system. Joints of an organism contain substances associated with degenerative osteoarthropathy, and are easy to obtain. Therefore, in this example, joints of swine were used to prepare the substance for treatment or prevention of degenerative osteoarthropathy by high-temperature carbonization.

Example 8: Clinical study with the substance prepared in Example 7 against degenerative osteoarthropathy

This example used the substance prepared in Example 7 for clinical studies, as specifically shown below.

1. Clinical data: 17 cases (in total) of patients with degenerative osteoarthropathy were enrolled, 4 males and 13 females, aged from 42 to 59.

2. Diagnosis criteria: The diagnosis criteria from *Diagnosis standards for bone damage diseases in traditional Chinese medicine.*

3. Treatment method: Patients orally took the substance for treatment of degenerative osteoarthropathy prepared in Example 7, 3 g per time, once a day, for 14 consecutive days.

4. Efficacy evaluation criteria: "Markedly effective" means that the patients' joint activities are not restricted in any way after treatment, and the clinical symptoms such as pain and discomfort have all disappeared. "Effective" means that after treatment the patients' pain has been relived, the restriction to join activity is reduced, and the discomfort and pain are lessened. "Ineffective" means that after treatment the patients' clinical symptoms have not been improved or even worsened, posing a serious impact on daily life.

$$\text{Total effective rate} = \text{markedly effective rate} + \text{effective rate}.$$

5. Efficacy results: Two months after the treatment the patients were observed for efficacy. Among the 17 patients in total, 12 showed "markedly effective", 4 showed "effective", and one showed "ineffective", with a total effective rate of 94.12%.

**Example** 9 Substance for treatment or prevention of asthma and method for preparing the substance

The main symptoms of asthma are wheezing and gasping, and the main pathogenesis of asthma is abnormal inhalation and exhalation of the lung caused by retained phlegm in the lung. The disease is located in the lung and mainly involves the lung meridian and the stomach meridian. Asthma is caused by the internal negatives and induced by external factors such that the lung qi is reversed upwards and the dispersing and descending function of the lung is impaired. People with pyretic pulmonary diseases feel unconscious first, then show vellus hair, aversion to wind and cold, yellow tongue, body fever, and heat conflicting which causes dyspnea with cough. From the perspective of traditional Chinese medicine, the cause of asthma lies in the lung. The lung of an organism contains substances associated with asthma, and is easy to obtain. Therefore, in this example, the lung of swine was used to prepare the substance for treatment or prevention of asthma by high-temperature carbonization. If other organs or tissue also contains asthma-associated pathologically changed substances, it is also preferable to use these asthma-associated pathologically changed organs or tissue to prepare the substance for treatment and/or prevention of asthma by high-temperature carbonization. In doing so, complex problems are simplified, and substances for treatment and/or prevention of asthma can be designed and prepared without knowing the specific pathologically changed structure or the mechanism of pathogenicity.

In this example, the lung of swine were used to prepare the substance for treatment or prevention of asthma by high-temperature carbonization.

**Example** 10: Clinical study with the substance prepared in Example 9 against asthma

This example used the substance prepared in Example 9 for efficacy evaluation, as specifically shown below.

1. Clinical data: 38 cases (in total) of outpatients and inpatients with asthma were enrolled, and randomly divided into a treatment group of 19 patients and a control group of 19 patients. There was no significant difference between the two groups in clinical data. There was no statistically significant difference between the two groups in age, course of disease, and complication (P>0.05), and they were comparable.

2. Diagnosis criteria: The diagnosis criteria for asthma from *Guidance for prevention and treatment of asthma in China (fundamental edition).*

3. Treatment method: Patients in the treatment group orally took the substance for treatment of asthma prepared in Example 9, 3 g per time, once a day, for 3 consecutive days. Patients in the control group inhaled the Salmeterol Xinafoate and Fluticasone Propionate powdery inhalant, 1 pack per time, twice a day, for 15 consecutive days.

4. Efficacy evaluation criteria: "Cured" means that after treatment, the clinical symptoms of asthma in the patients have disappeared completely without more onsets, and meanwhile the forced expiratory volume (FEV1) in the first second has increased by more than 35% as compared to that before the treatment or FEV1 is greater than 80% of the predicted value, and the peak expiratory flow (PEF) has a diurnal and nocturnal fluctuation rate less than 20%; "markedly effective" means that the clinical symptoms of asthma in patients after treatment have been significantly reduced as compared to those before treatment, but bronchodilators or glucocorticoids are still needed for controlling, and meanwhile FEV1 has increased by 25% to 35% as compared to that before treatment or FEV1 reaches 60%~79% of the predicted value, and PEF has a diurnal and nocturnal fluctuation rate greater than 20%; "effective" means that the clinical symptoms of asthma in patients after treatment have been reduced as compared to those before treatment, but bronchodilators or glucocorticoids are still needed for controlling, and meanwhile FEV1 has increased by 15% to 24% as compared to that before treatment; "ineffective" means that the clinical symptoms of asthma or the FEV1 value of patients have not been improved or even worsened after treatment.

$$\text{Total effective rate} = \text{cured rate} + \text{markedly effective rate} + \text{effective rate}.$$

5. Efficacy results: As shown in Table4, after treatment, in the control group 2 patients were "cured", 4 showed "markedly effective", 6 showed "effective", and 7 showed "ineffective", with a total effective rate of 63.16%, while in the treatment group 11 patients were "cured", 5 showed "markedly effective", 2 showed "effective", and 1 showed "ineffective", with a total effective rate as high as 94.74%. There was a statistically significant (P <0.05) difference between the two groups. During the treatment, no patient in the treatment group showed adverse effects or drug dependence, while 5 patients in the control group experienced adverse effects.

Table 4 Comparison of the effectiveness in patients with asthma after treatment

| Group | Number of patients | Cured | Markedly effective | Effective | Ineffective | Total effective rate (%) |
|---|---|---|---|---|---|---|
| Treatment | 19 | 11 | 5 | 2 | 1 | 94.74% |
| Control | 19 | 2 | 4 | 6 | 7 | 63.16% |

**Example** 11 Substance for treatment or prevention of allergic rhinitis and method for preparing the substance

Rhinitis is an inflammatory disease in the nasal cavity, which is an inflammation of the nasal mucosa caused by viruses, bacteria, allergens, various physical and chemical factors, and certain systemic diseases. Rhinitis caused by allergens is also called allergic rhinitis. As the course of the disease progresses, nasal mucosa congestion, swelling, exudation, hyperplasia, atrophy or necrosis can develop into chronic rhinitis. Therefore, from the perspective of Western medicine, the allergen that causes rhinitis includes substances associated with the disease. From the structural point of view, the root cause is the imbalance due to interactions between the biological structure system and the structure of the allergen. From the perspective of traditional Chinese medicine, "the lung opens up at the nose", and the lung communicates with the nature through the skin and hair in the nose and the mouth, and easily senses the invasion of foreign cold wind and air. In spring, there are many external winds that easily invade the head, face and skin surface. If the lung is weak at this time, the lung defense is compromised and the striae of the skin and muscle is loose, the wind often penetrates through the mouth and nose or the skin and hair to impede vascular circulation and cause blockage feelings, and the nasal orifices lose their patency and nourishment and become ill. If the lung is weak, the cold wind and "external negatives" can invade and cause nasal congestion, sneezing, and runny nose. If the wind enters from the nose and mouth, it will cause nasal congestion, runny nose and sticky nose. Therefore, according to the theory of traditional Chinese medicine, the cause of rhinitis is ascribed to the lungs. From a structural point of view, the root cause is the imbalance of some structures in the structure system of the lung of an organism, which leads to the structural imbalance between the biological structure system and the structure of the allergen. The balance of the biological structure system is dynamic and is associated with the external structural environment. For example, for humans, some substances, such as *Artemisia desertorum,* easily cause universal allergic rhinitis in humans, which means that the structural imbalance of the lung is a relative concept. For example, organisms such as swine, bovine, and Caprinae are not allergic to *Artemisia desertorum* and will not have allergic rhinitis. That is, the lung of swine, bovine and Caprinae contain structures that interact with *Artemisia desertorum* to avoid allergic rhinitis, i.e., structures that inhibit rhinitis.

The lung of an organism contains substances associated with allergic rhinitis and is readily available. Therefore, in this example, the lung of swine was selected and subjected to high-temperature carbonization to obtain the substance for treatment or prevention of allergic rhinitis.

**Example 12** Clinical application trial with the substance prepared in Example 11 for treatment of allergic rhinitis

This example used the substance prepared in Example 11 above for efficacy verification, as specifically shown below.

1. Clinical data: 60 cases (a total number) of 60 outpatients and inpatients with allergic rhinitis were enrolled, and were randomly divided into a treatment group with 30 patients and a control group with 30 patients. There was no significant difference between the two groups in clinical data and conditions. There was no statistically significant difference between the two groups in age, gender, course of disease, and complication (P>0.05), and they were comparable.

2. Diagnosis criteria: 2 or more clinical symptoms such as sneezing, clear water-like nasal discharge, nasal congestion, and nasal itching, appearing continuously or continually for 1 hour or more every day. It may be accompanied by eye symptoms such as eye itching and conjunctival hyperemia. Common signs often include pale nasal mucosa, edema, and watery nasal discharge. Positive in the allergen skin prick test.

3. Treatment method: the patients in the treatment group orally took the substance associated with treatment of allergic rhinitis prepared in Example 11, 3 g per time, once a day, for 3 consecutive days. The patients in the control group orally took loratadine 10 mg per time, once day, for 30 days.

4. Efficacy evaluation criteria: "cured" means that symptoms and signs disappear for 60 days without recurrence; "effective" means that symptoms and signs are alleviated, and the number of onsets is reduced; "ineffective" means that the effective standard is not met. Total effective rate = cured rate + effective rate.

5. Efficacy results: Table 5 shows that there were totally 28 patients in the treatment group showing "cured" and "effective" after treatment, with a total effective rate of 93.33%. In contrast, the control group showed a total effective rate of 66.67% after treatment, which is significantly lower (P<0.05). During the treatment, patients in the treatment group experienced no drug adverse reaction or drug dependence, and no recurrence during the 6-month follow-up, while adverse reactions occurred in 5 cases from in the control group and allergic symptoms appeared after contact with allergens after drug discontinuance.

Table 5 Comparison of effectiveness of treatment of patients with allergic rhinitis (as Examples)

| Group | Number of patients | Cured | Effective | Ineffective | Total effective rate (%) |
|---|---|---|---|---|---|
| Treatment | 30 | 22 | 6 | 2 | 93.33% |
| Control | 30 | 0 | 20 | 10 | 66.67% |

**Example 13**: Substance for treatment of conjunctivitis and method for preparing the substance

Conjunctivitis is redness or inflammation on the transparent membrane covering the eyeball and a part the membrane inside the inner eyelid. Allergens, chemical agents, and underlying diseases can cause redness of eyes. Therefore, from the perspective of Western medicine, the allergens that cause conjunctivitis are substances associated with conjunctivitis. From the structure point of view, the root cause is the imbalance between the biological structure system and the structure of the allergens upon interactions. From the perspective of traditional Chinese medicine, the *Neijing* says that "the liver is physiologically connected to the eyes" via the foot *Jueyin* channel, so the eyes are most closely associated with the liver meridian. Although "all meridians relate to the eyes" and "the essence of the five ZANG-organs and six FU-organs are all upwards converged on the eyes" indicating that the eyes have extensive connections with the meridians and viscera, it is most closely related to the foot *Jueyin* channel, and thus reference to "eyes" is always intimated with the foot *Jueyin.* If the liver blood is sufficient, the eyes will be bright, and the vision will be clear; if the liver blood is insufficient, the eyes will be short of nutrition, dry and dim, and the vision will be unclear or night blind; if the liver meridian is invaded by wind heat, the eyes will be red and itchy; if the liver has inflammation, the eyes are red and produce cataract; if the liver *Yang* is hyperactive, dizziness is felt in the head and eyes; if the liver wind is stirred, the eyes have squint and an upward vision. Therefore, from the perspective of traditional Chinese medicine, the cause of conjunctivitis lies in the liver. From a structural perspective, the root cause is imbalance of certain structures in the structure system of the liver of an organism, which leads to structural imbalance between the biological structure system and the allergen structure, resulting in conjunctivitis, especially allergic conjunctivitis. The balance of the biological structure system is dynamic and is related to the external structural environment. For example, for humans, some substances easily generally cause allergic conjunctivitis in humans, such as *Artemisia desertorum,* but not in organisms such as swine, bovine and Caprinae which are not allergic to *Artemisia desertorum,* which means that the structural imbalance of the liver is relative, and the liver structure of swine, bovine and Caprinae contains a structure that interacts with the structure of *Artemisia desertorum* to prevent allergic conjunctivitis in these organisms, i.e. a structure that inhibits the cause of conjunctivitis.

The liver of an organism contains conjunctivitis-associated substances, and is easy to obtain. Therefore, in this example, the liver of healthy swine was used to prepare the substance for treatment or prevention of conjunctivitis by high-temperature carbonization.

**Example 14**: Clinical study with the substance prepared in Example 13 against conjunctivitis

This example used the substance prepared in Example 13 for clinical studies, as specifically shown below.

1. Clinical data: 32 cases (in total) of outpatients and inpatients with conjunctivitis were enrolled. There was no significant difference between the two groups in clinical data. There was no statistically significant difference between the two groups in age, gender, course of disease, and complication (P>0.05), and they were comparable.

2. Diagnosis criteria: Foreign body sensation, burning sensation, a feel of heavy eyelids, and increased secretions in the affected eye; when the disease involves the cornea, photophobia, tearing, and varying degrees of vision loss may occur; conjunctival hyperemia; conjunctival edema; subconjunctival hemorrhage.

3. Treatment method: Patients orally took the substance for treatment of conjunctivitis prepared in Example 13, 3 g per time, once a day, for 2 consecutive days.

4. Efficacy evaluation criteria: "Cured" means complete disappearance of clinical symptoms without recurrence; "effective" means improvement of clinical symptoms; "ineffective" means no change in clinical symptoms or aggravation of clinical symptoms.

$$\text{Total effective rate} = \text{cured rate} + \text{effective rate}.$$

5. Efficacy results: After the treatment the patients were observed for efficacy. Among the 32 patients in total, 27 were "cured", 4 showed "effective", and one showed "ineffective", with a total effective rate of 96.88%.

**Example 15** Substance for treatment or prevention of chronic urticaria and method for preparing the substance

Urticaria is a skin disease characterized by wheal and angioedema, with various and complex causes. The foreign substances that cause urticaria such as castor beans, drugs and other substances are substance associated with diseases. Urticaria is a symptom, that is, an external manifestation of structural imbalance. Different substances can cause the same or similar structural imbalance, and the possible structures in these substances causing urticaria in an organism are the same or similar. Therefore, using only the stable structure corresponding to a known substance therein to act on the biological structure system can accomplish the supporting of *"the positive"* with *"the external negative"* according to the present application, thanks to the self-adapting, self-organizing, and structural stability-maintaining function of the biological structure system. According to the method for designing a substance for treatment and/or prevention of urticaria of the present disclosure, the stable structure corresponding to castor bean is preferably used in the present disclosure as the substance for treatment and/or prevention of urticaria. Because substances like ricin, ricinine, castor allergen and hemagglutinin in castor bean can cause urticaria with typical symptoms of disease, can be used as representative urticaria-associated substances and are also easy to obtain, in this example castor bean was selected and subjected to high-temperature carbonization to obtain the substance for treatment or prevention of urticaria.

**Example 16** Clinical study with the substance prepared in Example 15 against urticaria

This example used the substance prepared in Example 15 above for clinical studies, as specifically shown below.

1. Clinical data: A total of 56 outpatients and inpatients with urticaria were enrolled, and were randomly divided into a treatment group with 28 patients and a control group with 28 patients. There was no significant difference between the two groups in clinical data. There was no statistically significant difference between the two groups in age, gender, course of disease, and complication (P>0.05), and they were comparable.

2. Diagnosis criteria: The diagnosis criteria for urticaria from *Guidelines for diagnosis and treatment of urticaria in China (2014),* intermittent seizures, a course of disease $\geq$ 6 weeks.

3. Treatment method: the patients in the treatment group orally took the substance associated with treatment of urticaria prepared in Example 15, 3 g per time, once a day, for 3 consecutive days. The patients in the control group orally took loratadine 5 mg per time, once day, for 30 days.

4. Efficacy evaluation criteria: "Cured" means that wheal and pruritus have subsided, other accompanying symptoms and signs have disappeared, and re-exposure to the allergens will not produce allergic symptoms; "markedly effective" means that the pruritus has been significantly relieved, the wheal has subsided by 80% or more, and other accompanying symptoms and signs have been improved; "effective" means that the pruritus has been relieved, the wheal has subsided by 50% to 80%, and other accompanying symptoms and signs have been reduced; "ineffective" means that the clinical symptoms and signs have not changed significantly, or even worsened. Total effective rate = cured rate + markedly effective rate + effective rate.

5. Efficacy results: Table 6 shows that there were totally 26 patients in the treatment group showing "cured", "markedly effective" or "effective" after treatment, with a total effective rate of 92.86%. In contrast, the control group showed a total effective rate of 53.57% after treatment, which is significantly lower (P<0.05). During the treatment, patients experienced no adverse reaction or drug dependence, and no recurrence during the 6-month follow-up.

Table 6 Comparison of effectiveness of treatment of patients with chronic urticaria

| Group | Number of patients | Cured | Markedly effective | Effective | Ineffective | Total effective rate (%) |
|---|---|---|---|---|---|---|
| Treatment | 28 | 13 | 7 | 6 | 2 | 92.86% |
| Control | 28 | 0 | 9 | 6 | 13 | 53.57% |

Example 17 Substance for treatment and/or prevention of ankylosing spondylitis and methods for designing and preparing the substance

Ankylosing spondylitis is a chronic inflammatory disease whose main symptoms are sacroiliitis and spinal enthesitis, characterized by fibrosis and ossification of the large joints of the limbs, the annulus fibrosus disci intervertebralis and its nearby connective tissue, as well as ankylosis. The stable structure corresponding to the connective tissue is needed as an external structure to act on the organism suffering from ankylosing spondylitis to restore the balance of the structure system. Therefore, uthe stable structure corresponding to the connective tissue is used as a substance for treatment and/or prevention of ankylosing spondylitis. Because the connective tissue of an organism is easily available, in this example, the connective tissue of healthy bovine was selected and subjected to high-temperature carbonization to obtain the substance for treatment or prevention of ankylosing spondylitis.

Example 18: Clinical study with the substance prepared in Example 17 in patients with ankylosing spondylitis

1. Clinical data: This example enrolled totally 27 patients with ankylosing spondylitis, who were randomly divided into a treatment group of 14 patients and a control group of 13 patients according to the order of visit. The treatment group comprised 7 males and 7 females, 31-52 years old, with an average course of disease of 10.3 years. The control group comprised 6 males and 7 females, 33 to 51 years old, with an average course of disease of 10.8 years. There was no statistically significant difference between the two groups of patients in gender, age, condition and other clinical data (P>0.05), and they were comparable.

2. Diagnosis criteria: (1) lumbago and leg pain/discomfort of undetermined origin that occurred before the age of 40; (2) Insidious onset; (3) The course of disease >1 week; (4) Morning stiffness, static, or night pain, alleviated after exercise; (5) Positive for Gaenslen's test, percussion pain in sacroiliac joints and sacrum; (6) Inflammatory changes in the imaging examination of sacroiliac joints. Ankylosing spondylitis can be considered if one of the clinical criteria (1) to (4) is met on the basis of (5); and ankylosing spondylitis can be diagnosed if one of the clinical criteria is met on the basis of (6).

3. Treatment method: All patients were given diet and functional exercise guidance. Patients in the treatment group orally took the substance prepared in Example 17, 3 g per time, once a day, for 7 days. Patients in the control group orally took sulfasalazine enteric tablets, 3 tablets per time, 2 times a day, for 1 month.

4. Efficacy evaluation criteria: The total effective rate = (markedly effective + effective)/total number of patients* 100%. "Markedly effective" means that the patient's clinical symptoms and signs are significantly alleviated after treatment; "effective" means that the patient's clinical symptoms and signs are improved after treatment, but not to the standard level of "markedly effective"; "ineffective" means that the patient's clinical symptoms or signs are not improved after treatment or even become severe.

5. Treatment results: Both groups were treated for 1 month and observed for efficacy. Among the 14 patients in the treatment group, 12 showed "markedly effective", 1 showed "effective", and 1 showed "ineffective", with a total effective rate of 92.86%. Among the 13 patients in the control group, 2 cases showed "markedly effective", 5 showed "effective", and 6 showed "ineffective", with a total effective rate of 53.85%. The difference in the total effective rate between the treatment group and the control group was statistically significant (P<0.001), that is, the treatment group showed significantly better efficacy than the control group (see Table 7). During the treatment, patients in the treatment group did not show adverse drug reaction or drug dependence.

Table 7 Comparison of the effective rates between the two groups after treatment (as Examples)

| Group | Number of patients | Markedly effective | Effective | Ineffective | Total effective rate (%) |
|---|---|---|---|---|---|
| Treatment | 14 | 12 | 1 | 1 | 92.86% |
| Control | 13 | 2 | 5 | 6 | 53.85% |

**Example** 19 Substance for treatment or prevention of hypertension and methods for designing and preparing the substance

Most hypertensive patients, especially in the early stage of hypertension, show signs of increased sympathetic nerve activity: the release of catecholamines, epinephrine, and norepinephrine in the adrenal medulla increases, and the level of adrenaline in the blood continues to increase. Catecholamines act on the central nervous system, increase its excitability, enhance the release of norepinephrine from sympathetic nerve endings, increase cardiac output, and elevate the blood pressure. The content in the cranial cavity and the adrenal glands of an organism contain hypertension-associated pathologically changed substances, and are easy to obtain. Therefore, it is preferable to prepare the substance for treatment and/or prevention of hypertension by high-temperature carbonization of the content in the cranial cavity of an organism and/or the adrenal glands. If other organs or tissue also contains hypertension-associated pathologically changed substances, it is also preferable to use these hypertension-associated pathologically changed organs or tissue to prepare the substance for treatment and/or prevention of hypertension by high-temperature carbonization. In doing so, complex problems are simplified, and substances for treatment and/or prevention of hypertension can be designed and prepared without knowing the specific pathologically changed structure or the mechanism of pathogenicity. Therefore, in this example, content in the cranial cavity of Caprinae and the adrenal glands of swine were selected to prepare the substance for treatment or prevention of hypertension by high-temperature carbonization.

**Example** 20: Clinical study with the substance prepared in Example 19 against hypertension

This example used the substance prepared in Example 19 above for clinical studies, as specifically shown below.

1. Clinical data: In this example, totally 46 hypertensive patients were treated and observed clinically, including 25 males and 21 females, 18-75 years old, with a course of disease of 0.5 to 30 years, who were randomly divided into a treatment group of 23 patients and a control group of 23 patients. There was no significant difference between the two groups in clinical data. There was no statistically significant difference between the two groups in age, gender, course of disease, and complication (P>0.05), and they were comparable.

2. Diagnosis criteria: According to the *Guidelines for Diagnosis of Hypertension in China (2010),* hypertension can be diagnosed if the systolic pressure ≥140 mmHg and/or the diastolic pressure ≥90 mmHg. Grade 1 hypertension: systolic pressure 140 to 159 mmHg and/or diastolic pressure 90 to 99 mmHg; Grade 2 hypertension: systolic pressure 160 to 179 mmHg and/or diastolic pressure 100 to 109 mmHg; Grade 3 hypertension: systolic pressure ≥180 mmHg and/or diastolic pressure ≥110 mmHg.

3. Treatment method: Patients in the treatment group orally took the substance for treatment of hypertension prepared in Example 19, at a dose of 3 g, once a day, for 10 consecutive days. Patients in the control group orally took Levamlodipine Beslate and losartan potassium tablets, 3 times a day under supervision of a physician, and the dose was maintained for 3 months or more.

4. Efficacy evaluation criteria: "Cured" means that after stopping the drug the diastolic pressure is normal without recurrence; "markedly effective" means that the diastolic pressure drops by 10 mmHg or more and returns to normal, or the diastolic blood pressure does not return to normal but drops by 20 mmHg or more; "effective" means that the diastolic pressure drops by 10 mmHg or more, or the diastolic pressure drops by less than 10 mmHg and returns to normal, and the systolic pressure drops by 30 mmHg or more; "ineffective" means that the above standards are not met. Total effective rate = cured rate + markedly effective rate + effective rate.

5. Treatment results: After treatment, 23 hypertensive patients in the treatment group were tested for 3 months. As for the efficacy, 12 cases met the criteria for "cured", and 7 cases met the criteria for "markedly effective", see Table 8 below for details. The total effective rates of the treatment group and the control group were 95.65% and 56.52%, respectively, with a significant difference in efficacy between the two groups, indicating that the treatment group showed better results than the control group. During the treatment, in the control group there were 6 cases showing adverse drug reactions and 4 cases showing drug dependence. After administration, patients in the treatment group showing "effective" and "markedly effective" had their blood pressure well controlled, without drug dependence and adverse drug reactions.

Table 8 Comparison of the effective rates between the two groups after treatment (as Examples)

| Group | Number of patients | Cured | Markedly effective | Effective | Ineffective | Total effective rate (%) |
|---|---|---|---|---|---|---|
| Treatment | 23 | 12 | 7 | 3 | 1 | 95.65% |
| Control | 23 | 0 | 9 | 4 | 10 | 56.52% |

**Example 21** Substance for treatment or prevention of diabetes and method for preparing the substance

Diabetes is a syndrome of a series of metabolic disorders of proteins, fats, electrolytes, and the like caused by absolute or relative insufficient secretion of insulin and decreased sensitivity of target tissue cells to insulin. A variety of hormones secreted by the adrenal medulla affect glucose metabolism, antagonize the effect of insulin, and increase the blood sugar level. The substances in the islets of pancreas and/or adrenal glands of an organism contain diabetes-associated pathologically changed substances, and are easy to obtain. Therefore, it is preferable to prepare the substance for treatment and/or prevention of diabetes by high-temperature carbonization of the substances in the islets of pancreas and/or adrenal glands of an organism. If other organs or tissue also contains diabetes-associated pathologically changed substances, it is also preferable to use these diabetes-associated pathologically changed organs or tissue to prepare the substance for treatment and/or prevention of diabetes by high-temperature carbonization. In doing so, complex problems are simplified, and substances for treatment and/or prevention of diabetes can be designed and prepared without knowing the specific pathologically changed structure or the mechanism of pathogenicity. Therefore, in this example, the islets of pancreas and adrenal glands of swine were selected to prepare the substance for treatment or prevention of diabetes by high-temperature carbonization.

**Example** 22: Clinical study with the substance prepared in Example 21 against diabetes

This example used the substance prepared in Example 21 above for clinical studies, as specifically shown below.

1. Clinical data: In this example, 15 diabetic patients were treated and observed clinically, including 7 males and 8 females, 45-75 years old, with a course of disease of 0.5 to 10 years.

2. Diagnosis criteria: All patients met the 1999 WHO diagnostic criteria for diabetes: fasting blood glucose $\geq 7.0$ mmol/L, 2 hours postprandial or random blood glucose $\geq$ 11.1 mmol/L.

3. Treatment method: Patients orally took the substance for treatment of diabetes prepared in Example 21, at a dose of 3 g, once a day, for 7 consecutive days.

4. Efficacy evaluation criteria: The criteria were established with reference to the evaluation methods of efficacy against diabetes in the *Guiding Principles of Clinical Research on New Chinese Medicines for Treatment of Diabetes.* "Cured" means complete recovery of clinical symptoms and signs, with the fasting blood glucose and the 2 hours postprandial or random blood glucose returning to normal; "markedly effective" means that clinical symptoms and signs have been improved, with the fasting blood glucose $\leq$ 7.0 mmol/L, and the 2 h postprandial or random blood glucose $\leq$ 11.1 mmol/L; "effective" means that the clinical symptoms are better than those before treatment, and the blood glucose drops by more than 20% of the pre-treatment level, but does not reach the standard for "markedly effective"; "ineffective" means that the clinical symptoms and signs have not been improved or have been worsened, and the blood glucose indicators have not changed or have increased. Total effective rate = cured rate + markedly effective rate + effective rate.

5. Treatment results: After treatment, 15 diabetic patients were tested for 3 months. As for the efficacy, 4 cases met the criteria for "cured" with the blood glucose controlled at a normal level, 9 cases met the criteria for "markedly effective", and 2 cases showed "ineffective", with a total effective rate of 86.67%. After taking the medicine one time, the "cured", "markedly effective" and "effective" patients' blood glucose were well controlled during the follow-up of half a year, and there was no drug dependence and adverse drug reactions.

**Claims**

1. A method for designing a substance for treatment and/or prevention of diseases, comprising:

   using a stable structure corresponding to a substance associated with disease as the substance for treatment and/or prevention of the diseases;
   wherein the stable structure corresponding to the substance refers to the structure of the substance that is present in a solid state at a high temperature of 500°C or more.

2. The method of claim 1, wherein the stable structure corresponding to a substance associated with disease is one or more selected from:

   (1) a stable structure corresponding to a substance causing the disease in an organism;
   (2) a stable structure corresponding to a substance associated with the disease from an organism itself;
   (3) a stable structure corresponding to a substance containing a disease-inhibiting structure;
   (4) a stable structure corresponding to a substance containing the substance according to any one of (1) to (3) above.

3. A substance for treatment and/or prevention of diseases, designed according to the method of claim 1 or 2.

4. A method for preparing the substance for treatment and/or prevention of diseases of claim 3, wherein the stable structure corresponding to the substance is prepared by high-temperature carbonization.

5. A method for designing a substance for treatment and/or prevention of depression, comprising:

   using a stable structure corresponding to a depression-associated substance as the substance for treatment and/or prevention of depression;
   wherein the stable structure corresponding to the substance refers to the structure of the substance that is present in a solid state at a high temperature of 500°C or more.

6. The method of claim 6, wherein the stable structure corresponding to a depression-associated substance is one or more selected from:

   (1) a stable structure corresponding to a substance causing depression in an organism;
   (2) a stable structure corresponding to a substance of an organism itself associated with depression;
   (3) a stable structure corresponding to a substance containing a depression-inhibiting structure;
   (4) a stable structure corresponding to a substance containing the substance according to any one of (1) to (3) above.

7. The method of claim 6, wherein the depression-associated substance is selected from one or more of: substances in the nervous system, and substances in the endocrine system.

8. A substance for treatment and/or prevention of depression, designed according to the method of any one of claims 5-7.

9. A method for preparing the substance for treatment and/or prevention of depression of claim 8, wherein the stable structure corresponding to the depression-associated substance is prepared by high-temperature carbonization.

10. A method for designing a substance for treatment and/or prevention of anxiety, comprising:

    using a stable structure corresponding to an anxiety-associated substance as the substance for treatment and/or prevention of anxiety;
    wherein the stable structure corresponding to the substance refers to the structure of the substance that is present in a solid state at a high temperature of 500°C or more.

**11.** The method of claim 10, wherein the stable structure corresponding to an anxiety-associated substance is one or more selected from:

(1) a stable structure corresponding to a substance causing anxiety in an organism;
(2) a stable structure corresponding to a substance of an organism itself associated with anxiety;
(3) a stable structure corresponding to a substance containing an anxiety-inhibiting structure;
(4) a stable structure corresponding to a substance containing the substance according to any one of (1) to (3) above.

**12.** The method of claim 10, wherein the anxiety-associated substance is selected from one or more of: substances in the nervous system, and substances in the endocrine system.

**13.** A substance for treatment and/or prevention of anxiety, designed according to the method of any one of claims 10-12.

**14.** A method for preparing the substance for treatment and/or prevention of anxiety of claim 13, wherein the stable structure corresponding to the anxiety-associated substance is prepared by high-temperature carbonization.

**15.** A method for designing a substance for treatment and/or prevention of mania, comprising:

using a stable structure corresponding to a mania-associated substance as the substance for treatment and/or prevention of mania;
wherein the stable structure corresponding to the substance refers to the structure of the substance that is present in a solid state at a high temperature of 500°C or more.

**16.** The method of claim 15, wherein the stable structure corresponding to a mania-associated substance is one or more selected from:

(1) a stable structure corresponding to a substance causing mania in an organism;
(2) a stable structure corresponding to a substance of an organism itself associated with mania;
(3) a stable structure corresponding to a substance containing a mania-inhibiting structure;
(4) a stable structure corresponding to a substance containing the substance according to any one of (1) to (3) above.

**17.** The method of claim 15, wherein the mania-associated substance is selected from one or more of: substances in the nervous system, and substances in the endocrine system.

**18.** A substance for treatment and/or prevention of mania, designed according to the method of any one of claims 15-17.

**19.** A method for preparing the substance for treatment and/or prevention of mania of claim 18, wherein the stable structure corresponding to the mania-associated substance is prepared by high-temperature carbonization.

**20.** A method for designing a substance for treatment and/or prevention of bulimia, comprising:

using a stable structure corresponding to a bulimia-associated substance as the substance for treatment and/or prevention of bulimia;
wherein the stable structure corresponding to the substance refers to the structure of the substance that is present in a solid state at a high temperature of 500°C or more.

**21.** The method of claim 20, wherein the stable structure corresponding to a bulimia-associated substance is one or more selected from:

(1) a stable structure corresponding to a substance causing bulimia in an organism;
(2) a stable structure corresponding to a substance of an organism itself associated with bulimia;
(3) a stable structure corresponding to a substance containing a bulimia-inhibiting structure;
(4) a stable structure corresponding to a substance containing the substance according to any one of (1) to (3) above.

**22.** The method of claim 6, wherein the bulimia-associated substance is selected from one or more of: substances in the nervous system, and substances in the endocrine system.

23. A substance for treatment and/or prevention of bulimia, designed according to the method of any one of claims 20-22.

24. A method for preparing the substance for treatment and/or prevention of bulimia of claim 23, wherein the stable structure corresponding to the bulimia-associated substance is prepared by high-temperature carbonization.

25. A method for designing a substance for treatment and/or prevention of anorexia, comprising:

using a stable structure corresponding to an anorexia-associated substance as the substance for treatment and/or prevention of anorexia;
wherein the stable structure corresponding to the substance refers to the structure of the substance that is present in a solid state at a high temperature of 500°C or more.

26. The method of claim 25, wherein the stable structure corresponding to an anorexia-associated substance is one or more selected from:

(1) a stable structure corresponding to a substance causing anorexia in an organism;
(2) a stable structure corresponding to a substance of an organism itself associated with anorexia;
(3) a stable structure corresponding to a substance containing an anorexia-inhibiting structure;
(4) a stable structure corresponding to a substance containing the substance according to any one of (1) to (3) above.

27. The method of claim 25, wherein the anorexia-associated substance is selected from one or more of: substances in the nervous system, substances in the endocrine system, the ovary, and the intestinal tract of an organism.

28. A substance for treatment and/or prevention of anorexia, designed according to the method of any one of claims 25-27.

29. A method for preparing the substance for treatment and/or prevention of anorexia of claim 28, wherein the stable structure corresponding to the anorexia-associated substance is prepared by high-temperature carbonization.

30. A method for designing a substance for treatment and/or prevention of insomnia, comprising:

using a stable structure corresponding to an insomnia-associated substance as the substance for treatment and/or prevention of insomnia;
wherein the stable structure corresponding to the substance refers to the structure of the substance that is present in a solid state at a high temperature of 500°C or more.

31. The method of claim 30, wherein the stable structure corresponding to an insomnia-associated substance is one or more selected from:

(1) a stable structure corresponding to a substance causing insomnia in an organism;
(2) a stable structure corresponding to a substance of an organism itself associated with insomnia;
(3) a stable structure corresponding to a substance containing an insomnia-inhibiting structure;
(4) a stable structure corresponding to a substance containing the substance according to any one of (1) to (3) above.

32. The method of claim 31, wherein the insomnia-associated substance is selected from one or more of: substances in the nervous system, and substances in the endocrine system.

33. A substance for treatment and/or prevention of insomnia, designed according to the method of any one of claims 30-32.

34. A method for preparing the substance for treatment and/or prevention of insomnia of claim 33, wherein the stable structure corresponding to the insomnia-associated substance is prepared by high-temperature carbonization.

35. A method for designing a substance for treatment and/or prevention of tension headache, comprising:

using a stable structure corresponding to a tension headache-associated substance as the substance for treatment and/or prevention of tension headache;
wherein the stable structure corresponding to the substance refers to the structure of the substance that is present in a solid state at a high temperature of 500°C or more.

36. The method of claim 35, wherein the stable structure corresponding to a tension headache-associated substance is one or more selected from:

(1) a stable structure corresponding to a substance causing tension headache in an organism;
(2) a stable structure corresponding to a substance of an organism itself associated with tension headache;
(3) a stable structure corresponding to a substance containing a tension headache-inhibiting structure;
(4) a stable structure corresponding to a substance containing the substance according to any one of (1) to (3) above.

37. The method of claim 35, wherein the tension headache-associated substance is selected from one or more of: substances in the nervous system, and substances in the endocrine system.

38. A substance for treatment and/or prevention of tension headache, designed according to the method of any one of claims 35-37.

39. A method for preparing the substance for treatment and/or prevention of tension headache of claim 38, wherein the stable structure corresponding to the tension headache-associated substance is prepared by high-temperature carbonization.

40. A method for designing a substance for treatment and/or prevention of diabetes, comprising:

using a stable structure corresponding to a diabetes-associated substance as the substance for treatment and/or prevention of diabetes;
wherein the stable structure corresponding to the substance refers to the structure of the substance that is present in a solid state at a high temperature of 500°C or more.

41. The method of claim 40, wherein the stable structure corresponding to a diabetes-associated substance is one or more selected from:

(1) a stable structure corresponding to a substance causing diabetes in an organism;
(2) a stable structure corresponding to a substance of an organism itself associated with diabetes;
(3) a stable structure corresponding to a substance containing a diabetes-inhibiting structure;
(4) a stable structure corresponding to a substance containing the substance according to any one of (1) to (3) above.

42. The method of claim 40, wherein the diabetes-associated substance is derived from a secreting gland of an organism.

43. A substance for treatment and/or prevention of diabetes, designed according to the method of any one of claims 40-42.

44. A method for preparing the substance for treatment and/or prevention of diabetes of claim 43, wherein the stable structure corresponding to the diabetes-associated substance is prepared by high-temperature carbonization.

45. A method for designing a substance for treatment and/or prevention of hypertension, comprising:

using a stable structure corresponding to a hypertension-associated substance as the substance for treatment and/or prevention of hypertension;
wherein the stable structure corresponding to the substance refers to the structure of the substance that is present in a solid state at a high temperature of 500°C or more.

46. The method of claim 45, wherein the stable structure corresponding to a hypertension-associated substance is one or more selected from:

(1) a stable structure corresponding to a substance causing hypertension in an organism;

(2) a stable structure corresponding to a substance of an organism itself associated with hypertension;
(3) a stable structure corresponding to a substance containing a hypertension-inhibiting structure;
(4) a stable structure corresponding to a substance containing the substance according to any one of (1) to (3) above.

47. The method of claim 45, wherein the hypertension-associated substance is selected from one or more of: substances in the nervous system, substances in the endocrine system, viscera, and blood vessels of an organism.

48. A substance for treatment and/or prevention of hypertension, designed according to the method of any one of claims 45-47.

49. A method for preparing the substance for treatment and/or prevention of hypertension of claim 48, wherein the stable structure corresponding to the hypertension-associated substance is prepared by high-temperature carbonization.

50. A method for designing a substance for treatment and/or prevention of menoxenia, comprising:

using a stable structure corresponding to a menoxenia-associated substance as the substance for treatment and/or prevention of menoxenia;
wherein the stable structure corresponding to the substance refers to the structure of the substance that is present in a solid state at a high temperature of 500°C or more.

51. The method of claim 50, wherein the stable structure corresponding to a menoxenia-associated substance is one or more selected from:

(1) a stable structure corresponding to a substance causing menoxenia in an organism;
(2) a stable structure corresponding to a substance of an organism itself associated with menoxenia;
(3) a stable structure corresponding to a substance containing a menoxenia-inhibiting structure;
(4) a stable structure corresponding to a substance containing the substance according to any one of (1) to (3) above.

52. The method of claim 51, wherein the menoxenia-associated substance is selected from one or more of: substances in the nervous system, substances in the endocrine system, and the ovary.

53. A substance for treatment and/or prevention of menoxenia, designed according to the method of any one of claims 50-52.

54. A method for preparing the substance for treatment and/or prevention of menoxenia of claim 53, wherein the stable structure corresponding to the menoxenia-associated substance is prepared by high-temperature carbonization.

55. A method for designing a substance for treatment and/or prevention of amenorrhea, comprising:

using a stable structure corresponding to an amenorrhea-associated substance as the substance for treatment and/or prevention of amenorrhea;
wherein the stable structure corresponding to the substance refers to the structure of the substance that is present in a solid state at a high temperature of 500°C or more.

56. The method of claim 55, wherein the stable structure corresponding to an amenorrhea-associated substance is one or more selected from:

(1) a stable structure corresponding to a substance causing amenorrhea in an organism;
(2) a stable structure corresponding to a substance of an organism itself associated with amenorrhea;
(3) a stable structure corresponding to a substance containing an amenorrhea-inhibiting structure;
(4) a stable structure corresponding to a substance containing the substance according to any one of (1) to (3) above.

57. The method of claim 55, wherein the amenorrhea-associated substance is selected from one or more of: substances in the nervous system, substances in the endocrine system, and the ovary.

58. A substance for treatment and/or prevention of amenorrhea, designed according to the method of any one of claims 55-57.

59. A method for preparing the substance for treatment and/or prevention of amenorrhea of claim 58, wherein the stable structure corresponding to the amenorrhea-associated substance is prepared by high-temperature carbonization.

60. A method for designing a substance for treatment and/or prevention of dysmenorrhea, comprising:

using a stable structure corresponding to a dysmenorrhea-associated substance as the substance for treatment and/or prevention of dysmenorrhea;
wherein the stable structure corresponding to the substance refers to the structure of the substance that is present in a solid state at a high temperature of 500°C or more.

61. The method of claim 60, wherein the stable structure corresponding to a dysmenorrhea-associated substance is one or more selected from:

(1) a stable structure corresponding to a substance causing dysmenorrhea in an organism;
(2) a stable structure corresponding to a substance of an organism itself associated with dysmenorrhea;
(3) a stable structure corresponding to a substance containing a dysmenorrhea-inhibiting structure;
(4) a stable structure corresponding to a substance containing the substance according to any one of (1) to (3) above.

62. The method of claim 60, wherein the dysmenorrhea-associated substance is selected from one or more of: substances in the nervous system, substances in the endocrine system, and the ovary.

63. A substance for treatment and/or prevention of dysmenorrhea, designed according to the method of any one of claims 60-62.

64. A method for preparing the substance for treatment and/or prevention of dysmenorrhea of claim 63, wherein the stable structure corresponding to the dysmenorrhea-associated substance is prepared by high-temperature carbonization.

65. A method for designing a substance for treatment and/or prevention of stroke sequelae, comprising:

using a stable structure corresponding to a stroke sequelae-associated substance as the substance for treatment and/or prevention of stroke sequelae;
wherein the stable structure corresponding to the substance refers to the structure of the substance that is present in a solid state at a high temperature of 500°C or more.

66. The method of claim 65, wherein the stable structure corresponding to a stroke sequelae-associated substance is one or more selected from:

(1) a stable structure corresponding to a substance causing stroke sequelae in an organism;
(2) a stable structure corresponding to a substance of an organism itself associated with stroke sequelae;
(3) a stable structure corresponding to a substance containing a stroke sequelae-inhibiting structure;
(4) a stable structure corresponding to a substance containing the substance according to any one of (1) to (3) above.

67. The method of claim 65, wherein the stroke sequelae-associated substance is derived from substances in the cranial cavity of an organism.

68. A substance for treatment and/or prevention of stroke sequelae, designed according to the method of any one of claims 65-67.

69. A method for preparing the substance for treatment and/or prevention of stroke sequelae of claim 68, wherein the stable structure corresponding to the stroke sequelae-associated substance is prepared by high-temperature carbonization.

**70.** A method for designing a substance for treatment and/or prevention of lacrimation, comprising:

using a stable structure corresponding to a lacrimation-associated substance as the substance for treatment and/or prevention of lacrimation;

wherein the stable structure corresponding to the substance refers to the structure of the substance that is present in a solid state at a high temperature of 500°C or more.

**71.** The method of claim 70, wherein the stable structure corresponding to a lacrimation-associated substance is one or more selected from:

(1) a stable structure corresponding to a substance causing lacrimation in an organism;
(2) a stable structure corresponding to a substance of an organism itself associated with lacrimation;
(3) a stable structure corresponding to a substance containing a lacrimation-inhibiting structure;
(4) a stable structure corresponding to a substance containing the substance according to any one of (1) to (3) above.

**72.** The method of claim 70, wherein the lacrimation-associated substance is one or more selected from: eyes of an organism, immune organs of an organism, and the liver.

**73.** A substance for treatment and/or prevention of lacrimation, designed according to the method of any one of claims 70-72.

**74.** A method for preparing the substance for treatment and/or prevention of lacrimation of claim 73, wherein the stable structure corresponding to the lacrimation-associated substance is prepared by high-temperature carbonization.

**75.** A method for designing a substance for treatment and/or prevention of xerophthalmia, comprising:

using a stable structure corresponding to a xerophthalmia-associated substance as the substance for treatment and/or prevention of xerophthalmia;

wherein the stable structure corresponding to the substance refers to the structure of the substance that is present in a solid state at a high temperature of 500°C or more.

**76.** The method of claim 75, wherein the stable structure corresponding to a xerophthalmia-associated substance is one or more selected from:

(1) a stable structure corresponding to a substance causing xerophthalmia in an organism;
(2) a stable structure corresponding to a substance of an organism itself associated with xerophthalmia;
(3) a stable structure corresponding to a substance containing a xerophthalmia-inhibiting structure;
(4) a stable structure corresponding to a substance containing the substance according to any one of (1) to (3) above.

**77.** The method of claim 75, wherein the xerophthalmia-associated substance is one or more selected from: eyes of an organism, and the liver.

**78.** A substance for treatment and/or prevention of xerophthalmia, designed according to the method of any one of claims 75-77.

**79.** A method for preparing the substance for treatment and/or prevention of xerophthalmia of claim 78, wherein the stable structure corresponding to the xerophthalmia-associated substance is prepared by high-temperature carbonization.

**80.** A method for designing a substance for treatment and/or prevention of sexual dysfunction, comprising:

using a stable structure corresponding to a sexual dysfunction-associated substance as the substance for treatment and/or prevention of sexual dysfunction;

wherein the stable structure corresponding to the substance refers to the structure of the substance that is present in a solid state at a high temperature of 500°C or more.

**81.** The method of claim 80, wherein the stable structure corresponding to a sexual dysfunction-associated substance is one or more selected from:

(1) a stable structure corresponding to a substance causing sexual dysfunction in an organism;
(2) a stable structure corresponding to a substance of an organism itself associated with sexual dysfunction;
(3) a stable structure corresponding to a substance containing a sexual dysfunction-inhibiting structure;
(4) a stable structure corresponding to a substance containing the substance according to any one of (1) to (3) above.

**82.** The method of claim 80, wherein the sexual dysfunction-associated substance is one or more selected from: substances in the nervous system, substances in the endocrine system, and the reproductive organ of an organism.

**83.** A substance for treatment and/or prevention of sexual dysfunction, designed according to the method of any one of claims 80-82.

**84.** A method for preparing the substance for treatment and/or prevention of sexual dysfunction of claim 83, wherein the stable structure corresponding to the sexual dysfunction-associated substance is prepared by high-temperature carbonization.

**85.** A method for designing a substance for treatment and/or prevention of degenerative bone diseases, comprising:

using a stable structure corresponding to a degenerative bone disease associated substance as the substance for treatment and/or prevention of degenerative bone diseases;
wherein the stable structure corresponding to the substance refers to the structure of the substance that is present in a solid state at a high temperature of 500°C or more.

**86.** The method of claim 85, wherein the stable structure corresponding to a degenerative bone disease associated substance is one or more selected from:

(1) a stable structure corresponding to a substance causing degenerative bone diseases in an organism;
(2) a stable structure corresponding to a substance of an organism itself associated with degenerative bone diseases;
(3) a stable structure corresponding to a substance containing a degenerative bone diseases-inhibiting structure;
(4) a stable structure corresponding to a substance containing the substance according to any one of (1) to (3) above.

**87.** The method of claim 85, wherein the degenerative bone diseases associated substance is derived from the locomotor system.

**88.** A substance for treatment and/or prevention of degenerative bone diseases, designed according to the method of any one of claims 85-87.

**89.** A method for preparing the substance for treatment and/or prevention of degenerative bone diseases of claim 88, wherein the stable structure corresponding to the degenerative bone diseases associated substance is prepared by high-temperature carbonization.

**90.** A method for designing a substance for treatment and/or prevention of urticaria, comprising:

using a stable structure corresponding to an urticaria-associated substance as the substance for treatment and/or prevention of urticaria;
wherein the stable structure corresponding to the substance refers to the structure of the substance that is present in a solid state at a high temperature of 500°C or more.

**91.** The method of claim 90, wherein the stable structure corresponding to an urticaria-associated substance is one or more selected from:

(1) a stable structure corresponding to a substance causing urticaria in an organism;
(2) a stable structure corresponding to a substance of an organism itself associated with urticaria;

(3) a stable structure corresponding to a substance containing an urticaria-inhibiting structure;
(4) a stable structure corresponding to a substance containing the substance according to any one of (1) to (3) above.

**92.** A substance for treatment and/or prevention of urticaria, designed according to the method of any one of claims 90-91.

**93.** A method for preparing the substance for treatment and/or prevention of urticaria of claim 92, wherein the stable structure corresponding to the urticaria-associated substance is prepared by high-temperature carbonization.

**94.** A method for designing a substance for treatment and/or prevention of infectious diseases, comprising:

using a stable structure corresponding to an infectious disease associated substance as the substance for treatment and/or prevention of infectious diseases;
wherein the stable structure corresponding to the substance refers to the structure of the substance that is present in a solid state at a high temperature of 500°C or more.

**95.** The method of claim 94, wherein the stable structure corresponding to an infectious disease associated substance is one or more selected from:

(1) a stable structure corresponding to a substance causing infectious diseases in an organism;
(2) a stable structure corresponding to a substance of an organism itself associated with infectious diseases;
(3) a stable structure corresponding to a substance containing an infectious diseases-inhibiting structure;
(4) a stable structure corresponding to a substance containing the substance according to any one of (1) to (3) above.

**96.** The method of claim 94, wherein the infectious diseases associated substance is one or more selected from: bacteria, fungi, viruses, viroids, and parasites.

**97.** A substance for treatment and/or prevention of infectious diseases, designed according to the method of any one of claims 94-96.

**98.** A method for preparing the substance for treatment and/or prevention of infectious diseases of claim 97, wherein the stable structure corresponding to the infectious diseases associated substance is prepared by high-temperature carbonization.

**99.** A method for designing a substance for treatment and/or prevention of conjunctivitis, comprising:

using a stable structure corresponding to a conjunctivitis-associated substance as the substance for treatment and/or prevention of conjunctivitis;
wherein the stable structure corresponding to the substance refers to the structure of the substance that is present in a solid state at a high temperature of 500°C or more.

**100.** The method of claim 99, wherein the stable structure corresponding to a conjunctivitis-associated substance is one or more selected from:

(1) a stable structure corresponding to a substance causing conjunctivitis in an organism;
(2) a stable structure corresponding to a conjunctivitis-associated substance of an organism itself;
(3) a stable structure corresponding to a substance containing a conjunctivitis-inhibiting structure;
(4) a stable structure corresponding to a substance containing the substance according to any one of (1) to (3) above.

**101.** A substance for treatment and/or prevention of conjunctivitis, designed according to the method of any one of claims 99-100.

**102.** A method for preparing the substance for treatment and/or prevention of conjunctivitis of claim 101, wherein the stable structure corresponding to the conjunctivitis-associated substance is prepared by high-temperature carbonization.

**103.** A method for designing a substance for treatment and/or prevention of asthma, comprising:

using a stable structure corresponding to an asthma-associated substance as the substance for treatment and/or prevention of asthma;

wherein the stable structure corresponding to the substance refers to the structure of the substance that is present in a solid state at a high temperature of 500°C or more.

**104.** The method of claim 103, wherein the stable structure corresponding to an asthma-associated substance is one or more selected from:

(1) a stable structure corresponding to a substance causing asthma in an organism;

(2) a stable structure corresponding to an asthma-associated substance of an organism itself;

(3) a stable structure corresponding to a substance containing an asthma-inhibiting structure;

(4) a stable structure corresponding to a substance containing the substance according to any one of (1) to (3) above.

**105.** A substance for treatment and/or prevention of asthma, designed according to the method of any one of claims 103-104.

**106.** A method for preparing the substance for treatment and/or prevention of asthma of claim 105, wherein the stable structure corresponding to the asthma-associated substance is prepared by high-temperature carbonization.

**107.** A method for designing a substance for treatment and/or prevention of constipation, comprising:

using a stable structure corresponding to a constipation-associated substance as the substance for treatment and/or prevention of constipation;

wherein the stable structure corresponding to the substance refers to the structure of the substance that is present in a solid state at a high temperature of 500°C or more.

**108.** The method of claim 107, wherein the stable structure corresponding to a constipation-associated substance is one or more selected from:

(1) a stable structure corresponding to a substance causing constipation in an organism;

(2) a stable structure corresponding to a substance of an organism itself associated with constipation;

(3) a stable structure corresponding to a substance containing a constipation-inhibiting structure;

(4) a stable structure corresponding to a substance containing the substance according to any one of (1) to (3) above.

**109.** A substance for treatment and/or prevention of constipation, designed according to the method of any one of claims 107-108.

**110.** A method for preparing the above substance for treatment and/or prevention of constipation of claim 109, wherein the stable structure corresponding to the constipation-associated substance is prepared by high-temperature carbonization.

**111.** A method for designing a substance for treatment and/or prevention of ankylosing spondylitis, comprising:

using a stable structure corresponding to an ankylosing spondylitis-associated substance as the substance for treatment and/or prevention of ankylosing spondylitis;

wherein the stable structure corresponding to the substance refers to the structure of the substance that is present in a solid state at a high temperature of 500°C or more.

**112.** The method of claim 111, wherein the stable structure corresponding to an ankylosing spondylitis-associated substance is one or more selected from:

(1) a stable structure corresponding to a substance causing ankylosing spondylitis in an organism;

(2) a stable structure corresponding to a substance of an organism itself associated with ankylosing spondylitis;

(3) a stable structure corresponding to a substance containing an ankylosing spondylitis-inhibiting structure;

(4) a stable structure corresponding to a substance containing the substance according to any one of (1) to (3) above.

**113.** A substance for treatment and/or prevention of ankylosing spondylitis, designed according to the method of any one of claims 111-112.

**114.** A method for preparing the substance for treatment and/or prevention of ankylosing spondylitis of claim 113, wherein the stable structure corresponding to the ankylosing spondylitis-associated substance is prepared by high-temperature carbonization.

**115.** Use of the substance of any one of claims 3, 8, 13, 18, 23, 28, 33, 38, 43, 48, 53, 58, 63, 68, 73, 78, 83, 88, 92, 97, 101, 105, 109 and 113 in the manufacture of medicaments, health products, food, and food additives.